(19)

Europäisches Patentamt
European Patent Office
Office européen des brevets

(11) **EP 3 916 020 A1**

(12) **EUROPEAN PATENT APPLICATION**

(43) Date of publication:
01.12.2021 Bulletin 2021/48

(51) Int Cl.:
***C07K 16/40*** (2006.01)

(21) Application number: 21167802.4

(22) Date of filing: 30.03.2016

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2015 US 201562140289 P**
**30.03.2015 US 201562140277 P**
**04.09.2015 US 201562214293 P**

(62) Document number(s) of the earlier application(s) in accordance with Art. 76 EPC:
**16774069.5 / 3 286 226**

(71) Applicant: **Dyax Corp.**
**Lexington, MA 02421 (US)**

(72) Inventors:
• **CHYUNG, Yung**
**Lexington, 02421 (US)**

• **ADELMAN, Burt**
**Concord, 01742 (US)**
• **SEXTON, Daniel, J.**
**Melrose, 02176 (US)**

(74) Representative: **Carpmaels & Ransford LLP**
**One Southampton Row**
**London WC1B 5HA (GB)**

Remarks:
•This application was filed on 12-04-2021 as a divisional application to the application mentioned under INID code 62.
•Claims filed after the date of filing of the application / after the date of receipt of the divisional application (Rule 68(4) EPC).

(54) **PLASMA KALLIKREIN INHIBITORS AND USES THEREOF FOR PREVENTING HEREDITARY ANGIOEDEMA ATTACK**

(57) Provided herein are plasma kallikrein antibodies binding to active plasma kallikrein and methods of using such antibodies in preventing hereditary angioedema attack or reducing the rate of hereditary angioedema attack.

EP 3 916 020 A1

SEQUENCE LISTING

<110> Dyax Corp.

<120> PLASMA KALLIKREIN INHIBITORS AND USES THEREOF FOR PREVENTING
      HEREDITARY ANGIOEDEMA ATTACK

<130> D0617.70107WO00

<140> Not Yet Assigned
<141> 2016-03-30

<150> US 62/214,293
<151> 2015-09-24

<150> US 62/140,277
<151> 2015-03-30

<150> US 62/140,289
<151> 2015-03-30

<160> 10

<170> PatentIn version 3.5

<210> 1
<211> 469
<212> PRT
<213> Artificial Sequence

<220>
<223> Synthetic Polypeptide

<400> 1

Met Gly Trp Ser Cys Ile Leu Phe Leu Val Ala Thr Ala Thr Gly Ala
1               5                   10                  15


His Ser Glu Val Gln Leu Leu Glu Ser Gly Gly Gly Leu Val Gln Pro
            20                  25                  30


Gly Gly Ser Leu Arg Leu Ser Cys Ala Ala Ser Gly Phe Thr Phe Ser
            35                  40                  45


His Tyr Ile Met Met Trp Val Arg Gln Ala Pro Gly Lys Gly Leu Glu
        50                  55                  60


Trp Val Ser Gly Ile Tyr Ser Ser Gly Gly Ile Thr Val Tyr Ala Asp
65                  70                  75                  80


Ser Val Lys Gly Arg Phe Thr Ile Ser Arg Asp Asn Ser Lys Asn Thr
                85                  90                  95


Leu Tyr Leu Gln Met Asn Ser Leu Arg Ala Glu Asp Thr Ala Val Tyr
                100                 105                 110

```
Tyr Cys Ala Tyr Arg Arg Ile Gly Val Pro Arg Arg Asp Glu Phe Asp
        115             120             125

Ile Trp Gly Gln Gly Thr Met Val Thr Val Ser Ser Ala Ser Thr Lys
    130             135             140

Gly Pro Ser Val Phe Pro Leu Ala Pro Ser Ser Lys Ser Thr Ser Gly
145             150             155             160

Gly Thr Ala Ala Leu Gly Cys Leu Val Lys Asp Tyr Phe Pro Glu Pro
            165             170             175

Val Thr Val Ser Trp Asn Ser Gly Ala Leu Thr Ser Gly Val His Thr
        180             185             190

Phe Pro Ala Val Leu Gln Ser Ser Gly Leu Tyr Ser Leu Ser Ser Val
    195             200             205

Val Thr Val Pro Ser Ser Ser Leu Gly Thr Gln Thr Tyr Ile Cys Asn
    210             215             220

Val Asn His Lys Pro Ser Asn Thr Lys Val Asp Lys Arg Val Glu Pro
225             230             235             240

Lys Ser Cys Asp Lys Thr His Thr Cys Pro Pro Cys Pro Ala Pro Glu
            245             250             255

Leu Leu Gly Gly Pro Ser Val Phe Leu Phe Pro Pro Lys Pro Lys Asp
            260             265             270

Thr Leu Met Ile Ser Arg Thr Pro Glu Val Thr Cys Val Val Val Asp
        275             280             285

Val Ser His Glu Asp Pro Glu Val Lys Phe Asn Trp Tyr Val Asp Gly
    290             295             300

Val Glu Val His Asn Ala Lys Thr Lys Pro Arg Glu Glu Gln Tyr Asn
305             310             315             320

Ser Thr Tyr Arg Val Val Ser Val Leu Thr Val Leu His Gln Asp Trp
            325             330             335

Leu Asn Gly Lys Glu Tyr Lys Cys Lys Val Ser Asn Lys Ala Leu Pro
            340             345             350

Ala Pro Ile Glu Lys Thr Ile Ser Lys Ala Lys Gly Gln Pro Arg Glu
    355             360             365
```

32

```
Pro Gln Val Tyr Thr Leu Pro Pro Ser Arg Glu Glu Met Thr Lys Asn
    370                 375             380

Gln Val Ser Leu Thr Cys Leu Val Lys Gly Phe Tyr Pro Ser Asp Ile
385                 390             395                     400

Ala Val Glu Trp Glu Ser Asn Gly Gln Pro Glu Asn Asn Tyr Lys Thr
                405             410                 415

Thr Pro Pro Val Leu Asp Ser Asp Gly Ser Phe Phe Leu Tyr Ser Lys
            420             425             430

Leu Thr Val Asp Lys Ser Arg Trp Gln Gln Gly Asn Val Phe Ser Cys
        435             440             445

Ser Val Met His Glu Ala Leu His Asn His Tyr Thr Gln Lys Ser Leu
    450             455             460

Ser Leu Ser Pro Gly
465


<210>   2
<211>   231
<212>   PRT
<213>   Artificial Sequence

<220>
<223>   Synthetic Polypeptide

<400>   2

Met Gly Trp Ser Cys Ile Leu Phe Leu Val Ala Thr Ala Thr Gly Ala
1               5               10              15

His Ser Asp Ile Gln Met Thr Gln Ser Pro Ser Thr Leu Ser Ala Ser
            20              25                  30

Val Gly Asp Arg Val Thr Ile Thr Cys Arg Ala Ser Gln Ser Ile Ser
            35              40                  45

Ser Trp Leu Ala Trp Tyr Gln Gln Lys Pro Gly Lys Ala Pro Lys Leu
    50              55                  60

Leu Ile Tyr Lys Ala Ser Thr Leu Glu Ser Gly Val Pro Ser Arg Phe
65                  70              75                      80

Ser Gly Ser Gly Ser Gly Thr Glu Phe Thr Leu Thr Ile Ser Ser Leu
                85              90                  95
```

```
Gln Pro Asp Asp Phe Ala Thr Tyr Tyr Cys Gln Gln Tyr Asn Thr Tyr
        100                 105                 110

Trp Thr Phe Gly Gln Gly Thr Lys Val Glu Ile Lys Arg Thr Val Ala
        115                 120                 125

Ala Pro Ser Val Phe Ile Phe Pro Pro Ser Asp Glu Gln Leu Lys Ser
        130                 135                 140

Gly Thr Ala Ser Val Val Cys Leu Leu Asn Asn Phe Tyr Pro Arg Glu
145                 150                 155                 160

Ala Lys Val Gln Trp Lys Val Asp Asn Ala Leu Gln Ser Gly Asn Ser
                165                 170                 175

Gln Glu Ser Val Thr Glu Gln Asp Ser Lys Asp Ser Thr Tyr Ser Leu
        180                 185                 190

Ser Ser Thr Leu Thr Leu Ser Lys Ala Asp Tyr Glu Lys His Lys Val
        195                 200                 205

Tyr Ala Cys Glu Val Thr His Gln Gly Leu Ser Ser Pro Val Thr Lys
        210                 215                 220

Ser Phe Asn Arg Gly Glu Cys
225                 230


<210>   3
<211>   122
<212>   PRT
<213>   Artificial Sequence

<220>
<223>   Synthetic Polypeptide

<400>   3

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser His Tyr
            20                  25                  30

Ile Met Met Trp Val Arg Gln Ala Pro Gly Lys Gly Leu Glu Trp Val
            35                  40                  45

Ser Gly Ile Tyr Ser Ser Gly Gly Ile Thr Val Tyr Ala Asp Ser Val
        50                  55                  60

Lys Gly Arg Phe Thr Ile Ser Arg Asp Asn Ser Lys Asn Thr Leu Tyr
```

```
        65                   70                   75                   80

        Leu Gln Met Asn Ser Leu Arg Ala Glu Asp Thr Ala Val Tyr Tyr Cys
                        85                   90                   95

        Ala Tyr Arg Arg Ile Gly Val Pro Arg Arg Asp Glu Phe Asp Ile Trp
                        100                  105                  110

        Gly Gln Gly Thr Met Val Thr Val Ser Ser
                        115                  120


<210>   4
<211>   106
<212>   PRT
<213>   Artificial Sequence

<220>
<223>   Synthetic Polypeptide

<400>   4

Asp Ile Gln Met Thr Gln Ser Pro Ser Thr Leu Ser Ala Ser Val Gly
1                   5                   10                  15

Asp Arg Val Thr Ile Thr Cys Arg Ala Ser Gln Ser Ile Ser Ser Trp
                20                  25                  30

Leu Ala Trp Tyr Gln Gln Lys Pro Gly Lys Ala Pro Lys Leu Leu Ile
            35                  40                  45

Tyr Lys Ala Ser Thr Leu Glu Ser Gly Val Pro Ser Arg Phe Ser Gly
        50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asn Thr Tyr Trp Thr
                85                  90                  95

Phe Gly Gln Gly Thr Lys Val Glu Ile Lys
                100                  105


<210>   5
<211>   5
<212>   PRT
<213>   Artificial Sequence

<220>
<223>   Synthetic Polypeptide

<400>   5
```

His Tyr Ile Met Met
1               5


<210>   6
<211>   17
<212>   PRT
<213>   Artificial Sequence

<220>
<223>   Synthetic Polypeptide

<400>   6

Gly Ile Tyr Ser Ser Gly Gly Ile Thr Val Tyr Ala Asp Ser Val Lys
1               5                   10                  15


Gly


<210>   7
<211>   13
<212>   PRT
<213>   Artificial Sequence

<220>
<223>   Synthetic Polypeptide

<400>   7

Arg Arg Ile Gly Val Pro Arg Arg Asp Glu Phe Asp Ile
1               5                   10


<210>   8
<211>   11
<212>   PRT
<213>   Artificial Sequence

<220>
<223>   Synthetic Polypeptide

<400>   8

Arg Ala Ser Gln Ser Ile Ser Ser Trp Leu Ala
1               5                   10


<210>   9
<211>   7
<212>   PRT
<213>   Artificial Sequence

<220>
<223>   Synthetic Polypeptide

<400>   9

Lys Ala Ser Thr Leu Glu Ser
1               5

```
<210>  10
<211>  8
<212>  PRT
<213>  Artificial Sequence

<220>
<223>  Synthetic Polypeptide

<400>  10

Gln Gln Tyr Asn Thr Tyr Trp Thr
1                   5
```

**Description**

**RELATED APPLICATIONS**

This application claims the benefit under 35 U.S.C. § 119(e) of U.S. provisional application number 62/140,277, filed March 30, 2015, U.S. provisional application number 62/214,293, filed September 24, 2015 and U.S. provisional application number 62/140,289, filed March 30, 2015, the content of each of which is herein incorporated by reference in its entirety.

**BACKGROUND**

Plasma kallikrein is a serine protease component of the contact system and a potential drug target for different inflammatory, cardiovascular, infectious (sepsis) and oncology diseases (Sainz I.M. et al., Thromb Haemost 98, 77-83, 2007). The contact system is activated by either factor XIIa upon exposure to foreign or negatively charged surfaces or on endothelial cell surfaces by prolylcarboxypeptidases (Sainz I.M. et al., Thromb Haemost 98, 77-83, 2007). Activation of the plasma kallikrein amplifies intrinsic coagulation via its feedback activation of factor XII and enhances inflammation via the production of the proinflammatory nonapeptide bradykinin. As the primary kininogenase in the circulation, plasma kallikrein is largely responsible for the generation of bradykinin in the vasculature. A genetic deficiency in the C1-inhibitor protein (C1-INH), the major natural inhibitor of plasma kallikrein, leads to hereditary angioedema (HAE). Patients with HAE suffer from acute attacks of painful edema often precipitated by unknown triggers (Zuraw B.L. et al., N Engl J Med 359, 1027-1036, 2008).

**SUMMARY**

The present disclosure is, in part, based on the results derived from clinical studies showing that doses of DX-2930, an antibody binding to the active form of human plasma kallikrein, (e.g., 30 mg, 100 mg, 300 mg or 400 mg administered every two weeks) showed unexpected effectiveness in preventing HAE attack or reducing the rate of HAE attack in human patients. Further, DX-2930 treatment did not show evidence of dose-limiting toxicity when administered to humans. Overall, the results obtained from the instant study were unexpected since DX-2930 is the first completely specific plasma kallikrein inhibitor that has shown high efficacy in HAE treatment. This demonstrates that plasma kallikrein is central to disease pathogenesis.

Accordingly, one aspect of the present disclosure features a method of preventing HAE attack or reducing the rate of HAE attack (e.g., type I, II, or III HAE), the method comprising administering to a subject in need thereof an antibody binding to the active form of human plasma kallikrein (e.g., DX-2930) in an effective amount (e.g., about 30 mg -400 mg, about 100 mg - 400 mg, about 100 mg - 300 mg, or about 300 mg - 400 mg). In some embodiments, the antibody is administered every two to four weeks for at least two times. In some embodiments, the antibody is administered at 300 mg or 400 mg every 2 weeks to four weeks (e.g., every two weeks or every four weeks).

In any one of the methods described herein, the antibody can be administered by subcutaneous administration. In some embodiments, the subject is a human patient experiencing at least two HAE attack per year (e.g., at least one HAE attack within 6 month prior to the first administration, at least 2 HAE attacks within 3 months prior to the first administration, or at least 9 HAE attacks within 3 months prior to the first administration). The HAE can be type I HAE or type II HAE. For example, the method described herein is for prophylactic treatment of HAE.

The antibody used in any of the methods described herein may be an antibody (e.g., a full-length antibody or an antigen-binding fragment) that binds the same epitope as DX-2930 or competes against DX-2930 for binding to active human plasma kallikrein. In some embodiments, the antibody comprises the same heavy chain and light chain CDRs. In one example, the antibody is DX-2930. Any of the antibody as described herein (e.g., DX-2930) may be formulated in a pharmaceutical composition, which comprises a pharmaceutically acceptable carrier. In some examples, the pharmaceutical composition comprises sodium phosphate, citric acid, histidine, sodium chloride, and Tween 80. In one example, the antibody (e.g., DX-2930) is formulated in 30 mM sodium phosphate, 8.6 mM citric acid, 50 mM histidine, 90 mM sodium chloride, and 0.01% Tween 80, pH 6.0.

In yet other aspects, the present disclosure features a method of treating HAE (e.g., type I, II, or III), the method comprising administering to a subject in need thereof an antibody binding to the active form of human plasma kallikrein (e.g., DX-2930) in an effective amount (e.g., 100-400 mg, 100-300 mg, 150 mg or 300 mg), wherein the DX-2930 antibody is administered in a dosage regimen having a loading period (e.g., first administered every week such as for at least one week), a maintenance period (e.g., subsequently administered every two to four weeks), and optionally a follow-on period.

In some embodiments, the antibody is administered to the subject at 100 to 300 mg (e.g., 150 mg or 300 mg) during the loading period. The loading period may be two weeks. The antibody may be administered at day 0, day 7, and day 14 at, *e.g.,* 150 mg or 300 mg.

Alternatively or in addition, the antibody is administered to the subject at 100 to 300 mg (*e.g.,* 150 mg or 300 mg) during the maintenance period. The maintenance period may last for 10 weeks. The antibody can be administered at day 28, day 42, day 56, day 70 and day 84.

In any of the methods described herein, the method may further comprise administering to the subject the antibody after the maintenance period once every two to four weeks (*e.g.*, every two weeks or every four weeks). In some examples, the antibody is administered at 100 to 400 mg (*e.g.,* 100 mg to 300 mg, for example, 150 mg or 300 mg).

In some embodiments of any one of the methods described herein, the antibody can be administered by subcutaneous administration. In some embodiments, the subject is a human patient suffering from, suspected of having, or at risk for HAE attack. For example, the method described herein is for prophylactic treatment of HAE. The subject may be a human patient who experienced at least 2 attacks per year *(e.g.,* at least one attack per 4 weeks) prior to the treatment. In some embodiments, the antibody is administered for preventing HAE attack or for reducing the rate of HAE attack.

In some embodiments, the antibody (e.g., DX-2930) is first administered every week for one, two or three weeks and subsequently administered every two, three or four weeks. In some embodiments, the antibody (e.g., DX-2930) is subsequently administered every two weeks for ten weeks. In some embodiments, the subject has at least one attack every four weeks prior to the first administration.

The antibody to be used in any of the methods described herein may be an antibody that binds the same epitope as DX-2930 or competes against DX-2930 for binding to active human plasma kallikrein. In some embodiments, the antibody comprises the same heavy chain and light chain CDRs. In one example, the antibody is DX-2930.

Also within the scope of the present disclosure are (a) pharmaceutical compositions for use in treating HAE (e.g., preventing HAE attack or reducing the rate of HAE attack), the pharmaceutical composition comprising any of the anti-kallikrein antibodies described herein and a pharmaceutically acceptable carrier, wherein the pharmaceutical composition is administered to a subject following any of the treatment regimens described herein; and (b) use of the pharmaceutical composition for manufacturing a medicament for the treatment of HAE. Use of the antibodies for the intended purposes could be performed under the treating regimens as described herein.

The details of one or more embodiments of the invention are set forth in the description below. Other features or advantages of the present invention will be apparent from the following drawings and detailed description of several embodiments, and also from the appended claims.

**BRIEF DESCRIPTION OF DRAWINGS**

**FIGURE 1** shows DX-2930 plasma drug levels following subcutaneous dosing in HAE patients in the phase 1b study.

**FIGURE 2** shows fluorogenic activity assay of HAE patient samples from the phase 1b study.

**FIGURE 3** is a Western blot analysis of SCAT169 plasma from HAE patients from the phase 1b study.

**FIGURE 4** is a Western blot analysis of citrated plasma from HAE patients from the phase 1b study.

**FIGURE 5** is a Western blot analysis of citrated plasma activated ex vivo with FXIIa from HAE patients from the phase 1b study.

**FIGURE 6** shows the primary efficacy assessment period for patients in different dosing cohorts. **A.:** 300 mg cohort. **B.:** 400 mg cohort. Red bars show the interval assessed for efficacy.

**FIGURE 7** shows the reduction in HAE attack rate in patients treated with 300 mg, 400 mg, combined (300 and 400 mg) or placebo. Baseline was defined as historical HAE attacks over last 3 months prior to dosing. The data includes patients with a baseline rate of $\geq 2$ attacks in the last 3 months. Day 8 to 50 attack rates were unadjusted for baseline rates. Percent reduction in HAE attack rate over placebo and p-value were calculated based upon Mixed Model Repeated Measurements with Analysis of Variance (baseline attack rate as covariate) and assuming Poisson distribution..

**FIGURE 8** shows the incidence of HAE attacks in placebo-treated subjects. The X-axis shows the study day number.

**FIGURE 9** shows the mean DX-2930 concentration and HAE attack incidence following 30 mg dosage.

**FIGURE 10** shows the mean DX-2930 concentration and HAE attack incidence following 100 mg dosage.

**FIGURE 11** shows the mean DX-2930 concentration and HAE attack incidence following 300 mg dosage.

**FIGURE 12** shows the mean DX-2930 concentration and HAE attack incidence following 400 mg dosage. Excludes one patient who received only one dose (in order to derive the mean pharmacokinetic curve).

**FIGURE 13** shows DX-2930 concentration and HAE attacks in patients with historical attack rate $\geq 9$ attacks/3months. **A.:** a placebo patient. **B.:** a patient treated with 300 mg. and **C.-F.:** patients treated with 400 mg.

**FIGURE 14** shows an exemplary dosing regimen comprising a loading period and a maintenance period, which may be followed by an extended treatment period or a washout period.

## DETAILED DESCRIPTION

### Definitions

For convenience, before further description of the present invention, certain terms employed in the specification, examples and appended claims are defined here. Other terms are defined as they appear in the specification.

The singular forms "a", "an", and "the" include plural references unless the context clearly dictates otherwise.

The term "antibody" refers to a protein that includes at least one immunoglobulin variable domain (variable region) or immunoglobulin variable domain (variable region) sequence. For example, an antibody can include a heavy (H) chain variable region (abbreviated herein as VH or HV), and a light (L) chain variable region (abbreviated herein as VL or LV). In another example, an antibody includes two heavy (H) chain variable regions and two light (L) chain variable regions. The term "antibody" encompasses antigen-binding fragments of antibodies (e.g., single chain antibodies, Fab and sFab fragments, F(ab')$_2$, Fd fragments, Fv fragments, scFv, and domain antibodies (dAb) fragments (de Wildt et al., Eur J Immunol. 1996; 26(3):629-39)) as well as complete antibodies. An antibody can have the structural features of IgA, IgG, IgE, IgD, IgM (as well as subtypes thereof). Antibodies may be from any source, but primate (human and non-human primate) and primatized are preferred.

The VH and VL regions can be further subdivided into regions of hypervariability, termed "complementarity determining regions" ("CDRs"), interspersed with regions that are more conserved, termed "framework regions" ("FRs"). The extent of the framework region and CDRs have been defined (see, Kabat, E.A., et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917). Kabat definitions are used herein. Each VH and VL is typically composed of three CDRs and four FRs, arranged from amino-terminus to carboxy-terminus in the following order: FR1, CDR1, FR2, CDR2, FR3, CDR3, FR4.

As used herein, an "immunoglobulin variable domain sequence" refers to an amino acid sequence which can form the structure of an immunoglobulin variable domain such that one or more CDR regions are positioned in a conformation suitable for an antigen binding site. For example, the sequence may include all or part of the amino acid sequence of a naturally-occurring variable domain. For example, the sequence may omit one, two or more N- or C-terminal amino acids, internal amino acids, may include one or more insertions or additional terminal amino acids, or may include other alterations. In one embodiment, a polypeptide that includes immunoglobulin variable domain sequence can associate with another immunoglobulin variable domain sequence to form an antigen binding site, e.g., a structure that preferentially interacts with plasma kallikrein.

The $V_H$ or $V_L$ chain of the antibody can further include all or part of a heavy or light chain constant region, to thereby form a heavy or light immunoglobulin chain, respectively. In one embodiment, the antibody is a tetramer of two heavy immunoglobulin chains and two light immunoglobulin chains, wherein the heavy and light immunoglobulin chains are inter-connected by, e.g., disulfide bonds. In IgGs, the heavy chain constant region includes three immunoglobulin domains, CH1, CH2 and CH3. The light chain constant region includes a CL domain. The variable region of the heavy and light chains contains a binding domain that interacts with an antigen. The constant regions of the antibodies typically mediate the binding of the antibody to host tissues or factors, including various cells of the immune system (e.g., effector cells) and the first component (Clq) of the classical complement system. The light chains of the immunoglobulin may be of types kappa or lambda. In one embodiment, the antibody is glycosylated. An antibody can be functional for antibody-dependent cytotoxicity and/or complement-mediated cytotoxicity.

One or more regions of an antibody can be human or effectively human. For example, one or more of the variable regions can be human or effectively human. For example, one or more of the CDRs can be human, *e.g.,* HC CDR1, HC CDR2, HC CDR3, LC CDR1, LC CDR2, and/or LC CDR3. Each of the light chain (LC) and/or heavy chain (HC) CDRs can be human. HC CDR3 can be human. One or more of the framework regions can be human, e.g., FR1, FR2, FR3, and/or FR4 of the HC and/or LC. For example, the Fc region can be human. In one embodiment, all the framework regions are human, *e.g.,* derived from a human somatic cell, *e.g.,* a hematopoietic cell that produces immunoglobulins or a non-hematopoietic cell. In one embodiment, the human sequences are germline sequences, e.g., encoded by a germline nucleic acid. In one embodiment, the framework (FR) residues of a selected Fab can be converted to the amino-acid type of the corresponding residue in the most similar primate germline gene, especially the human germline gene. One or more of the constant regions can be human or effectively human. For example, at least 70, 75, 80, 85, 90, 92, 95, 98, or 100% of an immunoglobulin variable domain, the constant region, the constant domains (CH1, CH2, CH3, and/or CL1), or the entire antibody can be human or effectively human.

All or part of an antibody can be encoded by an immunoglobulin gene or a segment thereof. Exemplary human immunoglobulin genes include the kappa, lambda, alpha (IgA1 and IgA2), gamma (IgG1, IgG2, IgG3, IgG4), delta, epsilon and mu constant region genes, as well as the many immunoglobulin variable region genes. Full-length immunoglobulin "light chains" (about 25 KDa or about 214 amino acids) are encoded by a variable region gene at the NH2-terminus (about 110 amino acids) and a kappa or lambda constant region gene at the COOH-terminus. Full-length immunoglobulin "heavy chains" (about 50 KDa or about 446 amino acids), are similarly encoded by a variable region gene (about 116 amino acids) and one of the other aforementioned constant region genes, e.g., gamma (encoding about 330 amino acids). The length of human HC varies considerably because HC CDR3 varies from about 3 amino-acid residues to over 35 amino-acid residues.

The term "antigen-binding fragment" of a full length antibody refers to one or more fragments of a full-length antibody that retain the ability to specifically bind to a target of interest. Examples of binding fragments encompassed within the term "antigen-binding fragment" of a full length antibody and that retain functionality include (i) a Fab fragment, a monovalent fragment consisting of the VL, VH, CL and CH1 domains; (ii) a F(ab')2 fragment, a bivalent fragment including two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules known as single chain Fv (scFv). See, e.g., U.S. Pat. Nos. 5,260,203, 4,946,778, and 4,881,175; Bird et al. (1988) Science 242:423-426; and Huston et al. (1988) Proc. Natl. Acad. Sci. USA 85:5879-5883.

Antibody fragments can be obtained using any appropriate technique including conventional techniques known to those with skill in the art. The term "monospecific antibody" refers to an antibody that displays a single binding specificity and affinity for a particular target, e.g., epitope. This term includes a "monoclonal antibody" or "monoclonal antibody composition," which as used herein refers to a preparation of antibodies or fragments thereof of single molecular composition, irrespective of how the antibody was generated.

Antibodies are "germlined" by reverting one or more non-germline amino acids in framework regions to corresponding germline amino acids of the antibody, so long as binding properties are substantially retained.

The inhibition constant (Ki) provides a measure of inhibitor potency; it is the concentration of inhibitor required to reduce enzyme activity by half and is not dependent on enzyme or substrate concentrations. The apparent Ki (Ki,app) is obtained at different substrate concentrations by measuring the inhibitory effect of different concentrations of inhibitor (e.g., inhibitory binding protein) on the extent of the reaction (e.g., enzyme activity); fitting the change in pseudo-first order rate constant as a function of inhibitor concentration to the Morrison equation (Equation 1) yields an estimate of the apparent Ki value. The Ki is obtained from the y-intercept extracted from a linear regression analysis of a plot of Ki,app versus substrate concentration.

$$v = v_o - v_o \left( \frac{\left(K_{i,app} + I + E\right) - \sqrt{\left(K_{i,app} + I + E\right)^2 - 4 \cdot I \cdot E}}{2 \cdot E} \right)$$

Equation 1

Where v = measured velocity; v0 = velocity in the absence of inhibitor; Ki,app = apparent inhibition constant; I = total inhibitor concentration; and E = total enzyme concentration.

As used herein, "binding affinity" refers to the apparent association constant or KA. The KA is the reciprocal of the dissociation constant (KD). A binding antibody may, for example, have a binding affinity of at least 105, 106, 107, 108, 109, 1010 and 1011 M-1 for a particular target molecule, e.g., plasma kallikrein. Higher affinity binding of a binding antibody to a first target relative to a second target can be indicated by a higher KA (or a smaller numerical value KD) for binding the first target than the KA (or numerical value KD) for binding the second target. In such cases, the binding antibody has specificity for the first target (e.g., a protein in a first conformation or mimic thereof) relative to the second target (e.g., the same protein in a second conformation or mimic thereof; or a second protein). Differences in binding affinity (e.g., for specificity or other comparisons) can be at least 1.5, 2, 3, 4, 5, 10, 15, 20, 37.5, 50, 70, 80, 91, 100, 500, 1000, 10,000 or 105 fold.

Binding affinity can be determined by a variety of methods including equilibrium dialysis, equilibrium binding, gel filtration, ELISA, surface plasmon resonance, or spectroscopy (e.g., using a fluorescence assay). Exemplary conditions for evaluating binding affinity are in HBS-P buffer (10 mM HEPES pH7.4, 150 mM NaCl, 0.005% (v/v) Surfactant P20). These techniques can be used to measure the concentration of bound and free binding protein as a function of binding protein (or target) concentration. The concentration of bound binding protein ([Bound]) is related to the concentration of free binding protein ([Free]) and the concentration of binding sites for the binding protein on the target where (N) is the number of binding sites per target molecule by the following equation:

$$[Bound] = N \cdot [Free]/((1/KA) + [Free]).$$

It is not always necessary to make an exact determination of KA, though, since sometimes it is sufficient to obtain a quantitative measurement of affinity, *e.g.,* determined using a method such as ELISA or FACS analysis, is proportional to KA, and thus can be used for comparisons, such as determining whether a higher affinity is, e.g., 2 fold higher, to obtain a qualitative measurement of affinity, or to obtain an inference of affinity, *e.g.,* by activity in a functional assay, *e.g.,* an *in vitro* or *in vivo* assay.

The term "binding antibody" (or "binding protein" used interchangeably herein) refers to an antibody that can interact with a target molecule. This term is used interchangeably with "ligand." A "plasma kallikrein binding antibody" refers to an antibody that can interact with (e.g., bind) plasma kallikrein, and includes, in particular, antibodies that preferentially or specifically interact with and/or inhibit plasma kallikrein. An antibody inhibits plasma kallikrein if it causes a decrease in the activity of plasma kallikrein as compared to the activity of plasma kallikrein in the absence of the antibody and under the same conditions.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (*e.g.*, glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g.*, threonine, valine, isoleucine) and aromatic side chains (*e.g.*, tyrosine, phenylalanine, tryptophan, histidine).

It is possible for one or more framework and/or CDR amino acid residues of a binding protein to include one or more mutations (*e.g.,* substitutions (*e.g.,* conservative substitutions or substitutions of non-essential amino acids), insertions, or deletions) relative to a binding protein described herein. A plasma kallikrein binding protein may have mutations (*e.g.,* substitutions (*e.g.,* conservative substitutions or substitutions of non-essential amino acids), insertions, or deletions) (*e.g.,* at least one, two, three, or four, and/or less than 15, 12, 10, 9, 8, 7, 6, 5, 4, 3, or 2 mutations) relative to a binding protein described herein, e.g., mutations which do not have a substantial effect on protein function. The mutations can be present in framework regions, CDRs, and/or constant regions. In some embodiments, the mutations are present in a framework region. In some embodiments, the mutations are present in a CDR. In some embodiments, the mutations are present in a constant region. Whether or not a particular substitution will be tolerated, i.e., will not adversely affect biological properties, such as binding activity, can be predicted, e.g., by evaluating whether the mutation is conservative or by the method of Bowie, et al. (1990) Science 247:1306-1310.

An "effectively human" immunoglobulin variable region is an immunoglobulin variable region that includes a sufficient number of human framework amino acid positions such that the immunoglobulin variable region does not elicit an immunogenic response in a normal human. An "effectively human" antibody is an antibody that includes a sufficient number of human amino acid positions such that the antibody does not elicit an immunogenic response in a normal human.

An "epitope" refers to the site on a target compound that is bound by a binding protein (e.g., an antibody such as a Fab or full length antibody). In the case where the target compound is a protein, the site can be entirely composed of amino acid components, entirely composed of chemical modifications of amino acids of the protein (e.g., glycosyl moieties), or composed of combinations thereof. Overlapping epitopes include at least one common amino acid residue, glycosyl group, phosphate group, sulfate group, or other molecular feature.

A first binding antibody "binds to the same epitope" as a second binding antibody if the first binding antibody binds to the same site on a target compound that the second binding antibody binds, or binds to a site that overlaps (*e.g.,* 50%, 60%, 70%, 80%, 90%, or 100% overlap, *e.g.,* in terms of amino acid sequence or other molecular feature (e.g., glycosyl group, phosphate group, or sulfate group)) with the site that the second binding antibody binds.

A first binding antibody "competes for binding" with a second binding antibody if the binding of the first binding antibody to its epitope decreases (e.g., by 10%, 20%, 30%, 40%, 50%, 60%, 70%, 80%, 90%, 100%, or more) the amount of the second binding antibody that binds to its epitope. The competition can be direct (*e.g.,* the first binding antibody binds to an epitope that is the same as, or overlaps with, the epitope bound by the second binding antibody), or indirect (*e.g.,* the binding of the first binding antibody to its epitope causes a steric change in the target compound that decreases the ability of the second binding antibody to bind to its epitope).

Calculations of "homology" or "sequence identity" between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). The optimal alignment is determined as the best score using the GAP program in the GCG software package with a Blossum 62 scoring matrix with a gap penalty of 12, a gap extend penalty of 4, and a frameshift gap penalty of 5. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences.

In a preferred embodiment, the length of a reference sequence aligned for comparison purposes is at least 30%, preferably at least 40%, more preferably at least 50%, even more preferably at least 60%, and even more preferably at least 70%, 80%, 90%, 92%, 95%, 97%, 98%, or 100% of the length of the reference sequence. For example, the reference sequence may be the length of the immunoglobulin variable domain sequence.

A "humanized" immunoglobulin variable region is an immunoglobulin variable region that is modified to include a sufficient number of human framework amino acid positions such that the immunoglobulin variable region does not elicit an immunogenic response in a normal human. Descriptions of "humanized" immunoglobulins include, for example, U.S. 6,407,213 and U.S. 5,693,762.

An "isolated" antibody refers to an antibody that is removed from at least 90% of at least one component of a natural sample from which the isolated antibody can be obtained. Antibodies can be "of at least" a certain degree of purity if the species or population of species of interest is at least 5, 10, 25, 50, 75, 80, 90, 92, 95, 98, or 99% pure on a weight-weight basis.

A "patient," "subject" or "host" (these terms are used interchangeably) to be treated by the subject method may mean either a human or non-human animal.

The terms "prekallikrein" and "preplasma kallikrein" are used interchangeably herein and refer to the zymogen form of active plasma kallikrein, which is also known as prekallikrein.

As used herein, the term "substantially identical" (or "substantially homologous") is used herein to refer to a first amino acid or nucleic acid sequence that contains a sufficient number of identical or equivalent (e.g., with a similar side chain, e.g., conserved amino acid substitutions) amino acid residues or nucleotides to a second amino acid or nucleic acid sequence such that the first and second amino acid or nucleic acid sequences have (or encode proteins having) similar activities, e.g., a binding activity, a binding preference, or a biological activity. In the case of antibodies, the second antibody has the same specificity and has at least 50%, at least 25%, or at least 10% of the affinity relative to the same antigen.

Sequences similar or homologous (e.g., at least about 85% sequence identity) to the sequences disclosed herein are also part of this application. In some embodiments, the sequence identity can be about 85%, 90%, 91%, 92%, 93%, 94%, 95%, 96%, 97%, 98%, 99% or higher. In some embodiments, a plasma kallikrein binding antibody can have about 85%, 90%, 91%, 92%, 93%, 94%, 95%, 96%, 97%, 98%, 99% or higher sequence identity to an antibody described herein. In some embodiments, a plasma kallikrein binding antibody can have about 85%, 90%, 91%, 92%, 93%, 94%, 95%, 96%, 97%, 98%, 99% or higher sequence identity in the HC and/or LC framework regions (*e.g.*, HC and/or LC FR 1, 2, 3, and/or 4) to an antibody described herein (e.g., DX-2930). In some embodiments, a plasma kallikrein binding antibody can have about 85%, 90%, 91%, 92%, 93%, 94%, 95%, 96%, 97%, 98%, 99% or higher sequence identity in the HC and/or LC CDRs (*e.g.,* HC and/or LC CDR1, 2, and/or 3) to an antibody described herein (e.g., DX-2930). In some embodiments, a plasma kallikrein binding antibody can have about 85%, 90%, 91%, 92%, 93%, 94%, 95%, 96%, 97%, 98%, 99% or higher sequence identity in the constant region (*e.g.,* CH1, CH2, CH3, and/or CL1) to an antibody described herein (*e.g.*, DX-2930).

In addition, substantial identity exists when the nucleic acid segments hybridize under selective hybridization conditions (e.g., highly stringent hybridization conditions), to the complement of the strand. The nucleic acids may be present in whole cells, in a cell lysate, or in a partially purified or substantially pure form.

Statistical significance can be determined by any art known method. Exemplary statistical tests include: the Students T-test, Mann Whitney U non-parametric test, and Wilcoxon non-parametric statistical test. Some statistically significant relationships have a P value of less than 0.05 or 0.02. Particular binding proteins may show a difference, *e.g.,* in specificity or binding that are statistically significant (e.g., P value < 0.05 or 0.02). The terms "induce", "inhibit", "potentiate", "elevate", "increase", "decrease" or the like, *e.g.,* which denote distinguishable qualitative or quantitative differences between two states, may refer to a difference, e.g., a statistically significant difference, between the two states.

A "therapeutically effective dosage" preferably modulates a measurable parameter, e.g., plasma kallikrein activity, by a statistically significant degree or at least about 20%, more preferably by at least about 40%, even more preferably by at least about 60%, and still more preferably by at least about 80% relative to untreated subjects. The ability of a compound to modulate a measurable parameter, *e.g.,* a disease-associated parameter, can be evaluated in an animal model system predictive of efficacy in human disorders and conditions. Alternatively, this property of a composition can be evaluated by examining the ability of the compound to modulate a parameter in vitro.

The term "treating" as used herein refers to the application or administration of a composition including one or more active agents to a subject, who has an allergic disease, a symptom of the allergic disease, or a predisposition toward the allergic disease, with the purpose to cure, heal, alleviate, relieve, alter, remedy, ameliorate, improve, or affect the disease, the symptoms of the disease, or the predisposition toward the disease. "Prophylactic treatment," also known as "preventive treatment," refers to a treatment that aims at protecting a person from, or reducing the risk for a disease to which he or she has been, or may be, exposed.

The term "preventing" a disease in a subject refers to subjecting the subject to a pharmaceutical treatment, e.g., the administration of a drug, such that at least one symptom of the disease is prevented, that is, administered prior to clinical manifestation of the unwanted condition (e.g., disease or other unwanted state of the host animal) so that it protects the host against developing the unwanted condition. "Preventing" a disease may also be referred to as "prophylaxis" or "prophylactic treatment."

A "prophylactically effective amount" refers to an amount effective, at dosages and for periods of time necessary, to achieve the desired prophylactic result. Typically, because a prophylactic dose is used in subjects prior to or at an earlier stage of disease, the prophylactically effective amount will be less than the therapeutically effective amount.

**Antibodies Specific to Plasma Kallikrein**

Plasma kallikrein binding antibodies for use in the methods described herein can be full-length (e.g., an IgG (e.g., an IgG1, IgG2, IgG3, IgG4), IgM, IgA (e.g., IgA1, IgA2), IgD, and IgE) or can include only an antigen-binding fragment (e.g., a Fab, F(ab')2 or scFv fragment. The binding antibody can include two heavy chain immunoglobulins and two light chain immunoglobulins, or can be a single chain antibody. Plasma kallikrein binding antibodies can be recombinant proteins such as humanized, CDR grafted, chimeric, deimmunized, or in vitro generated antibodies, and may optionally include constant regions derived from human germline immunoglobulin sequences. In one embodiment, the plasma kallikrein binding antibody is a monoclonal antibody.

In one aspect, the disclosure features an antibody (e.g., an isolated antibody) that binds to plasma kallikrein (e.g., human plasma kallikrein and/or murine kallikrein) and includes at least one immunoglobulin variable region. For example, the antibody includes a heavy chain (HC) immunoglobulin variable domain sequence and/or a light chain (LC) immunoglobulin variable domain sequence. In one embodiment, the antibody binds to and inhibits plasma kallikrein, e.g., human plasma kallikrein and/or murine kallikrein.

The antibody can include one or more of the following characteristics: (a) a human CDR or human framework region; (b) the HC immunoglobulin variable domain sequence comprises one or more (e.g., 1, 2, or 3) CDRs that are at least 85, 88, 89, 90, 91, 92, 93, 94, 95, 96, 97, 98, 99, or 100% identical to a CDR of a HC variable domain described herein; (c) the LC immunoglobulin variable domain sequence comprises one or more (e.g., 1, 2, or 3) CDRs that are at least 85, 88, 89, 90, 91, 92, 93, 94, 95, 96, 97, 98, 99, or 100% identical to a CDR of a LC variable domain described herein; (d) the LC immunoglobulin variable domain sequence is at least 85, 88, 89, 90, 91, 92, 93, 94, 95, 96, 97, 98, 99, or 100% identical to a LC variable domain described herein (e.g., overall or in framework regions or CDRs); (e) the HC immunoglobulin variable domain sequence is at least 85, 88, 89, 90, 91, 92, 93, 94, 95, 96, 97, 98, 99, or 100% identical to a HC variable domain described herein (e.g., overall or in framework regions or CDRs); (f) the antibody binds an epitope bound by an antibody described herein, or competes for binding with an antibody described herein; (g) a primate CDR or primate framework region; (h) the HC immunoglobulin variable domain sequence comprises a CDR1 that differs by at least one amino acid but by no more than 2 or 3 amino acids from the CDR1 of a HC variable domain described herein; (i) the HC immunoglobulin variable domain sequence comprises a CDR2 that differs by at least one amino acid but by no more than 2, 3, 4, 5, 6, 7, or 8 amino acids from the CDR2 of a HC variable domain described herein; (j) the HC immunoglobulin variable domain sequence comprises a CDR3 that differs by at least one amino acid but by no more than 2, 3, 4, 5, or 6 amino acids from the CDR3 of a HC variable domain described herein; (k) the LC immunoglobulin variable domain sequence comprises a CDR1 that differs by at least one amino acid but by no more than 2, 3, 4, or 5 amino acids from the CDR1 of a LC variable domain described herein; (1) the LC immunoglobulin variable domain sequence comprises a CDR2 that differs by at least one amino acid but by no more than 2, 3, or 4 amino acids from the CDR2 of a LC variable domain described herein; (m) the LC immunoglobulin variable domain sequence comprises a CDR3 that differs by at least one amino acid but by no more than 2, 3, 4, or 5 amino acids from the CDR3 of a LC variable domain described herein ; (n) the LC immunoglobulin variable domain sequence differs by at least one amino acid but by no more than 2, 3, 4, 5, 6, 7, 8, 9, or 10 amino acids from a LC variable domain described herein (e.g., overall or in framework regions or CDRs); and (o) the HC immunoglobulin variable domain sequence differs by at least one amino acid but by no more than 2, 3, 4, 5, 6, 7, 8, 9, or 10 amino acids from a HC variable domain described herein (e.g., overall or in framework regions or CDRs).

The plasma kallikrein binding protein may be an isolated antibody (e.g., at least 70, 80, 90, 95, or 99% free of other proteins). In some embodiments, the plasma kallikrein binding antibody, or composition thereof, is isolated from antibody cleavage fragments (e.g., DX-2930) that are inactive or partially active (e.g., bind plasma kallikrein with a Ki, app of 5000 nM or greater) compared to the plasma kallikrein binding antibody. For example, the plasma kallikrein binding antibody is at least 70% free of such antibody cleavage fragments; in other embodiments the binding antibody is at least 80%, at least 90%, at least 95%, at least 99% or even 100% free from antibody cleavage fragments that are inactive or partially active.

The plasma kallikrein binding antibody may additionally inhibit plasma kallikrein, e.g., human plasma kallikrein.

In some embodiments, the plasma kallikrein binding antibody does not bind prekallikrein (e.g., human prekallikrein and/or murine prekallikrein), but binds to the active form of plasma kallikrein (e.g., human plasma kallikrein and/or murine kallikrein).

In certain embodiments, the antibody binds at or near the active site of the catalytic domain of plasma kallikrein, or a fragment thereof, or binds an epitope that overlaps with the active site of plasma kallikrein.

In some aspects, the antibody binds the same epitope or competes for binding with an antibody described herein.

The antibody can bind to plasma kallikrein, e.g., human plasma kallikrein, with a binding affinity of at least $10^5$, $10^6$, $10^7$, $10^8$, $10^9$, $10^{10}$ and $10^{11}$ M$^{-1}$. In one embodiment, the antibody binds to human plasma kallikrein with a $K_{off}$ slower than $1 \times 10^{-3}$, $5 \times 10^{-4}$ s$^{-1}$, or $1 \times 10^{-4}$ s$^{-1}$. In one embodiment, the antibody binds to human plasma kallikrein with a $K_{on}$ faster than $1 \times 10^2$, $1 \times 10^3$, or $5 \times 10^3$ M$^{-1}$s$^{-1}$. In one embodiment, the antibody binds to plasma kallikrein, but does not bind to tissue kallikrein and/or plasma prekallikrein (e.g., the antibody binds to tissue kallikrein and/or plasma prekallikrein less effectively (*e.g.,* 5-, 10-, 50-, 100-, or 1000-fold less or not at all, *e.g.,* as compared to a negative control) than it binds to plasma kallikrein.

In one embodiment, the antibody inhibits human plasma kallikrein activity, *e.g.,* with a Ki of less than $10^{-5}$, $10^{-6}$, $10^{-7}$, $10^{-8}$, $10^{-9}$, and $10^{-10}$ M. The antibody can have, for example, an $IC_{50}$ of less than 100 nM, 10 nM, 1, 0.5, or 0.2 nM. For example, the antibody may modulate plasma kallikrein activity, as well as the production of Factor XIIa *(e.g.,* from Factor XII) and/or bradykinin (*e.g.,* from high-molecular-weight kininogen (HMWK)). The antibody may inhibit plasma kallikrein activity, and/or the production of Factor XIIa (*e.g.,* from Factor XII) and/or bradykinin (e.g., from high-molecular-weight kininogen (HMWK)). The affinity of the antibody for human plasma kallikrein can be characterized by a $K_D$ of less than 100 nm, less than 10 nM, less than 5 nM, less than 1 nM, less than 0.5 nM. In one embodiment, the antibody inhibits plasma kallikrein, but does not inhibit tissue kallikrein (e.g., the antibody inhibits tissue kallikrein less effectively (e.g., 5-, 10-, 50-, 100-, or 1000-fold less or not at all, *e.g.,* as compared to a negative control) than it inhibits plasma kallikrein.

In some embodiments, the antibody has an apparent inhibition constant ($K_{i,app}$) of less than 1000, 500, 100, 5, 1, 0.5 or 0.2 nM.

Plasma kallikrein binding antibodies may have their HC and LC variable domain sequences included in a single polypeptide (e.g., scFv), or on different polypeptides (*e.g.,* IgG or Fab).

In one embodiment, the HC and LC variable domain sequences are components of the same polypeptide chain. In another, the HC and LC variable domain sequences are components of different polypeptide chains. For example, the antibody is an IgG, *e.g.,* IgG1, IgG2, IgG3, or IgG4. The antibody can be a soluble Fab. In other implementations the antibody includes a Fab2', scFv, minibody, scFv::Fc fusion, Fab::HSA fusion, HSA::Fab fusion, Fab::HSA::Fab fusion, or other molecule that comprises the antigen combining site of one of the binding proteins herein. The VH and VL regions of these Fabs can be provided as IgG, Fab, Fab2, Fab2', scFv, PEGylated Fab, PEGylated scFv, PEGylated Fab2, VH::CH1::HSA+LC, HSA::VH::CH1+LC, LC::HSA + VH::CH1, HSA::LC + VH::CH1, or other appropriate construction.

In one embodiment, the antibody is a human or humanized antibody or is non-immunogenic in a human. For example, the antibody includes one or more human antibody framework regions, *e.g.,* all human framework regions, or framework regions at least 85, 88, 89, 90, 91, 92, 93, 94, 95, 96, 97, 98, 99% identical to human framework regions. In one embodiment, the antibody includes a human Fc domain, or an Fc domain that is at least 95, 96, 97, 98, or 99% identical to a human Fc domain.

In one embodiment, the antibody is a primate or primatized antibody or is non-immunogenic in a human. For example, the antibody includes one or more primate antibody framework regions, e.g., all primate framework regions, or framework regions at least 85, 88, 89, 90, 91, 92, 93, 94, 95, 96, 97, 98, 99% identical to primate framework regions. In one embodiment, the antibody includes a primate Fc domain, or an Fc domain that is at least 95, 96, 97, 98, or 99% identical to a primate Fc domain. "Primate" includes humans (*Homo sapiens*), chimpanzees (*Pan troglodytes* and *Pan paniscus* (bonobos)), gorillas (*Gorilla gorilla*)*,* gibons, monkeys, lemurs, aye-ayes (Daubentonia madagascariensis), and tarsiers.

In some embodiments, the affinity of the primate antibody for human plasma kallikrein is characterized by a $K_D$ of less than 1000, 500, 100, 10, 5, 1, 0.5 nM, e.g., less than 10 nM, less than 1 nM, or less than 0.5 nM.

In certain embodiments, the antibody includes no sequences from mice or rabbits (e.g., is not a murine or rabbit antibody).

In some embodiments, the antibody used in the methods described herein may be DX-2930 as described herein or a functional variant thereof, or an antibody that binds the same epitope as DX-2930 or competes against DX-2930 for binding to active plasma kallikrein.

In one example, a functional variant of DX-2930 comprises the same complementary determining regions (CDRs) as DX-2930, as determined by the same method. In another example, the functional variants of DX-2930 may contain one or more mutations (e.g., conservative substitutions) in the FRs of either the $V_H$ or the $V_L$ as compared to those in the $V_H$ and $V_L$ of DX-2930. Preferably, such mutations do not occur at residues which are predicted to interact with one or more of the CDRs, which can be determined by routine technology. In other embodiments, the functional variants described herein contain one or more mutations (e.g., 1, 2, or 3) within one or more of the CDR regions of DX-2930. Preferably, such functional variants retain the same regions/residues responsible for antigen-binding as the parent. In yet other embodiments, a functional variant of DX-2930 may comprise a $V_H$ chain that comprises an amino acid sequence at least 85% (e.g., 90%, 92%, 94%, 95%, 96%, 97%, 98%, or 99%) identical to that of the $V_H$ of DX-2930 and/or a $V_L$ chain that has an amino acid sequence at least 85% (e.g., 90%, 92%, 94%, 95%, 96%, 97%, 98%, or 99%) identical to that of the $V_L$ of DX-2930. These variants are capable of binding to the active form of plasma kallikrein and preferably do not bind to prekallikrein.

The "percent identity" of two amino acid sequences is determined using the algorithm of Karlin and Altschul Proc. Natl. Acad. Sci. USA 87:2264-68, 1990, modified as in Karlin and Altschul Proc. Natl. Acad. Sci. USA 90:5873-77, 1993. Such an algorithm is incorporated into the NBLAST and XBLAST programs (version 2.0) of Altschul, et al. J. Mol. Biol. 215:403-10, 1990. BLAST protein searches can be performed with the XBLAST program, score=50, wordlength=3 to obtain amino acid sequences homologous to the protein molecules of interest. Where gaps exist between two sequences, Gapped BLAST can be utilized as described in Altschul et al., Nucleic Acids Res. 25(17):3389-3402, 1997. When utilizing BLAST and Gapped BLAST programs, the default parameters of the respective programs (e.g., XBLAST and NBLAST) can be used.

In some embodiments, the antibody used in the methods and compositions described herein may be the DX-2930 antibody. The heavy and light chain full and variable sequences for DX-2930 are provided below, with signal sequences in italics. The CDRs are boldfaced and underlined (based on the Kabat numbering scheme).

DX-2930 Heavy Chain Amino Acid Sequence (451 amino acids, 49439.02 Da)

*MGWSCILFLVATATGAHS*EVQLLESGGGLVQPGGSLRLSCAASGFTFS**HYIMM**
WVRQAPGKGLEWVS**GIYSSGGITVYADSVKG**RFTISRDNSKNTLYLQMNSL
RAEDTAVYYCAY**RRIGVPRRDEFDI**WGQGTMVTVSSASTKGPSVFPLAPSSK
STSGGTAALGCLVKDYFPEPVTVSWNSGALTSGVHTFPAVLQSSGLYSLSSVV

TVPSSSLGTQTYICNVNHKPSNTKVDKRVEPKSCDKTHTCPPCPAPELLGGPS
VFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPG
(SEQ ID NO: 1)

DX-2930 Light Chain Amino Acid Sequence (213 amino acids, 23419.08 Da)

*MGWSCILFLVATATGAHS*DIQMTQSPSTLSASVGDRVTITC**RASQSISSWLA**WY
QQKPGKAPKLLIY**KASTLES**GVPSRFSGSGSGTEFTLTISSLQPDDFATYYC**QQ**
**YNTYWT**FGQGTKVEIKRTVAAPSVFIFPPSDEQLKSGTASVVCLLNNFYPREA
KVQWKVDNALQSGNSQESVTEQDSKDSTYSLSSTLTLSKADYEKHKVYACE
VTHQGLSSPVTKSFNRGEC (SEQ ID NO: 2)

DX-2930 Heavy Chain Variable Domain Amino Acid Sequence

EVQLLESGGGLVQPGGSLRLSCAASGFTFS**HYIMM**WVRQAPGKGLEWVS**GI**
**YSSGGITVYADSVKG**RFTISRDNSKNTLYLQMNSLRAEDTAVYYCAY**RRIG**
**VPRRDEFDI**WGQGTMVTVSS (SEQ ID NO: 3)

DX-2930 Light Chain Variable Domain Amino Acid Sequence

DIQMTQSPSTLSASVGDRVTITC**RASQSISSWLA**WYQQKPGKAPKLLIY**KAST
LES**GVPSRFSGSGSGTEFTLTISSLQPDDFATYYC**QQYNTYWT**FGQGTKVEIK
(SEQ ID NO: 4)

Table 1. CDRs for DX-2930.

| CDR | Amino acid sequence |
| --- | --- |
| Heavy chain CDR1 | HYIMM (SEQ ID NO: 5) |
| Heavy chain CDR2 | GIYSSGGITVYADSVKG (SEQ ID NO: 6) |
| Heavy chain CDR3 | RRIGVPRRDEFDI (SEQ ID NO: 7) |
| Light chain CDR1 | RASQSISSWLA (SEQ ID NO: 8) |
| Light chain CDR2 | KASTLES (SEQ ID NO: 9) |
| Light chain CDR3 | QQYNTYWT (SEQ ID NO: 10) |

**Antibody Preparation**

An antibody as described herein (e.g., DX-2930) can be made by any method known in the art. See, for example, Harlow and Lane, (1988) Antibodies: A Laboratory Manual, Cold Spring Harbor Laboratory, New York and Greenfield, (2013) Antibodies: A Laboratory Manual, Second edition, Cold Spring Harbor Laboratory Press.

The sequence encoding the antibody of interest, e.g., DX-2930, may be maintained in vector in a host cell and the host cell can then be expanded and frozen for future use. In an alternative, the polynucleotide sequence may be used for genetic manipulation to "humanize" the antibody or to improve the affinity (affinity maturation), or other characteristics of the antibody. For example, the constant region may be engineered to more resemble human constant regions to avoid immune response if the antibody is used in clinical trials and treatments in humans. It may be desirable to genetically manipulate the antibody sequence to obtain greater affinity to the target antigen and greater efficacy in inhibiting the activity of PKal. It will be apparent to one of skill in the art that one or more polynucleotide changes can be made to the antibody and still maintain its binding specificity to the target antigen.

In other embodiments, fully human antibodies can be obtained by using commercially available mice that have been engineered to express specific human immunoglobulin proteins. Transgenic animals that are designed to produce a more desirable (e.g., fully human antibodies) or more robust immune response may also be used for generation of humanized or human antibodies. Examples of such technology are Xenomouse[RTM] from Amgen, Inc. (Fremont, Calif.) and HuMAb-Mouse[RTM] and TC Mouse™ from Medarex, Inc. (Princeton, N.J.). In another alternative, antibodies may be made recombinantly by phage display or yeast technology. See, for example, U.S. Pat. Nos. 5,565,332; 5,580,717; 5,733,743; and 6,265,150; and Winter et al., (1994) Annu. Rev. Immunol. 12:433-455. Alternatively, the phage display technology (McCafferty et al., (1990) Nature 348:552-553) can be used to produce human antibodies and antibody fragments in vitro, from immunoglobulin variable (V) domain gene repertoires from unimmunized donors.

Antigen-binding fragments of an intact antibody (full-length antibody) can be prepared via routine methods. For example, F(ab')2 fragments can be produced by pepsin digestion of an antibody molecule, and Fab fragments that can be generated by reducing the disulfide bridges of F(ab')2 fragments.

Genetically engineered antibodies, such as humanized antibodies, chimeric antibodies, single-chain antibodies, and bi-specific antibodies, can be produced via, e.g., conventional recombinant technology. In one example, DNA encoding a monoclonal antibodies specific to a target antigen can be readily isolated or synthesized. The DNA may be placed into one or more expression vectors, which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. See, e.g., PCT Publication No. WO 87/04462. The DNA can then be modified, for example, by substituting the coding sequence for human heavy and light chain constant domains in place of the homologous murine sequences, Morrison et al., (1984) Proc. Nat. Acad. Sci. 81:6851, or by covalently joining to the immunoglobulin coding sequence all or part of the coding sequence for a non-immunoglobulin polypeptide. In that manner, genetically engineered antibodies, such as "chimeric" or "hybrid" antibodies; can be prepared that have the binding specificity of a target antigen.

Techniques developed for the production of "chimeric antibodies" are well known in the art. See, e.g., Morrison et al. (1984) Proc. Natl. Acad. Sci. USA 81, 6851; Neuberger et al. (1984) Nature 312, 604; and Takeda et al. (1984) Nature 314:452.

Methods for constructing humanized antibodies are also well known in the art. See, e.g., Queen et al., Proc. Natl. Acad. Sci. USA, 86:10029-10033 (1989). In one example, variable regions of VH and VL of a parent non-human antibody are subjected to three-dimensional molecular modeling analysis following methods known in the art. Next, framework amino acid residues predicted to be important for the formation of the correct CDR structures are identified using the same molecular modeling analysis. In parallel, human VH and VL chains having amino acid sequences that are homologous to those of the parent non-human antibody are identified from any antibody gene database using the parent VH and VL sequences as search queries. Human VH and VL acceptor genes are then selected.

The CDR regions within the selected human acceptor genes can be replaced with the CDR regions from the parent non-human antibody or functional variants thereof. When necessary, residues within the framework regions of the parent chain that are predicted to be important in interacting with the CDR regions (see above description) can be used to substitute for the corresponding residues in the human acceptor genes.

A single-chain antibody can be prepared via recombinant technology by linking a nucleotide sequence coding for a heavy chain variable region and a nucleotide sequence coding for a light chain variable region. Preferably, a flexible linker is incorporated between the two variable regions. Alternatively, techniques described for the production of single chain antibodies (U.S. Patent Nos. 4,946,778 and 4,704,692) can be adapted to produce a phage or yeast scFv library and scFv clones specific to a PKal can be identified from the library following routine procedures. Positive clones can be subjected to further screening to identify those that inhibits PKal activity.

Some antibodies, e.g., Fabs, can be produced in bacterial cells, *e.g., E. coli* cells (see e.g., Nadkarni, A. et al., 2007 Protein Expr Purif 52(1):219-29). For example, if the Fab is encoded by sequences in a phage display vector that includes a suppressible stop codon between the display entity and a bacteriophage protein (or fragment thereof), the vector nucleic acid can be transferred into a bacterial cell that cannot suppress a stop codon. In this case, the Fab is not fused to the gene III protein and is secreted into the periplasm and/or media.

Antibodies can also be produced in eukaryotic cells. In one embodiment, the antibodies *(e.g.,* scFv's) are expressed in a yeast cell such as *Pichia* (see, *e.g.,* Powers et al., 2001, J. Immunol. Methods. 251:123-35; Schoonooghe S. et al., 2009 BMC Biotechnol. 9:70; Abdel-Salam, HA. et al., 2001 Appl Microbiol Biotechnol 56(1-2):157-64; Takahashi K. et al., 2000 Biosci Biotechnol Biochem 64(10):2138-44; Edqvist, J. et al., 1991 J Biotechnol 20(3):291-300), *Hanseula,* or *Saccharomyces.* One of skill in the art can optimize antibody production in yeast by optimizing, for example, oxygen conditions (see e.g., Baumann K., et al. 2010 BMC Syst. Biol. 4:141), osmolarity (see e.g., Dragosits, M. et al., 2010 BMC Genomics 11:207), temperature (see e.g., Dragosits, M. et al., 2009 J Proteome Res. 8(3):1380-92), fermentation conditions (see e.g., Ning, D. et al. 2005 J. Biochem. and Mol. Biol. 38(3): 294-299), strain of yeast (see e.g., Kozyr, AV et al. 2004 Mol Biol (Mosk) 38(6):1067-75; Horwitz, AH. et al., 1988 Proc Natl Acad Sci U S A 85(22):8678-82; Bowdish, K. et al. 1991 J Biol Chem 266(18):11901-8), overexpression of proteins to enhance antibody production (see e.g., Gasser, B. et al., 2006 Biotechol. Bioeng. 94(2):353-61), level of acidity of the culture (see e.g., Kobayashi H., et al., 1997 FEMS Microbiol Lett 152(2):235-42), concentrations of substrates and/or ions (see e.g., Ko JH. et al., 2996 Appl Biochem Biotechnol 60(1):41-8). In addition, yeast systems can be used to produce antibodies with an extended half-life (see e.g., Smith, BJ. et al. 2001 Bioconjug Chem 12(5):750-756),

In one preferred embodiment, antibodies are produced in mammalian cells. Preferred mammalian host cells for expressing the clone antibodies or antigen-binding fragments thereof include Chinese Hamster Ovary (CHO cells) (including dhfr- CHO cells, described in Urlaub and Chasin, 1980, Proc. Natl. Acad. Sci. USA 77:4216-4220, used with a DHFR selectable marker, e.g., as described in Kaufman and Sharp, 1982, Mol. Biol. 159:601 621), lymphocytic cell lines, e.g., NS0 myeloma cells and SP2 cells, COS cells, HEK293T cells (J. Immunol. Methods (2004) 289(1-2):65-80), and a cell from a transgenic animal, e.g., a transgenic mammal. For example, the cell is a mammary epithelial cell.

In some embodiments, plasma kallikrein binding antibodies are produced in a plant or cell-free based system (see e.g., Galeffi, P., et al., 2006 J Transl Med 4:39).

In addition to the nucleic acid sequence encoding the diversified immunoglobulin domain, the recombinant expression vectors may carry additional sequences, such as sequences that regulate replication of the vector in host cells (e.g., origins of replication) and selectable marker genes. The selectable marker gene facilitates selection of host cells into which the vector has been introduced (see e.g., U.S. Patent Nos. 4,399,216, 4,634,665 and 5,179,017). For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in *dhfr⁻* host cells with methotrexate selection/amplification) and the *neo* gene (for G418 selection).

In an exemplary system for recombinant expression of an antibody, or antigen-binding portion thereof, a recombinant expression vector encoding both the antibody heavy chain and the antibody light chain is introduced into *dhfr⁻* CHO cells by calcium phosphate-mediated transfection. Within the recombinant expression vector, the antibody heavy and light chain genes are each operatively linked to enhancer/promoter regulatory elements (e.g., derived from SV40, CMV, adenovirus and the like, such as a CMV enhancer/AdMLP promoter regulatory element or an SV40 enhancer/AdMLP promoter regulatory element) to drive high levels of transcription of the genes. The recombinant expression vector also carries a DHFR gene, which allows for selection of CHO cells that have been transfected with the vector using methotrexate selection/amplification. The selected transformant host cells are cultured to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium. For example, some antibodies can be isolated by affinity chromatography with a Protein A or Protein G coupled matrix.

For antibodies that include an Fc domain, the antibody production system may produce antibodies in which the Fc region is glycosylated. For example, the Fc domain of IgG molecules is glycosylated at asparagine 297 in the CH2 domain. This asparagine is the site for modification with biantennary-type oligosaccharides. It has been demonstrated that this glycosylation is required for effector functions mediated by Fcg receptors and complement C1q (Burton and Woof, 1992, Adv. Immunol. 51:1-84; Jefferis et al., 1998, Immunol. Rev. 163:59-76). In one embodiment, the Fc domain is produced in a mammalian expression system that appropriately glycosylates the residue corresponding to asparagine 297. The Fc domain can also include other eukaryotic post-translational modifications.

Antibodies can also be produced by a transgenic animal. For example, U.S. Pat. No. 5,849,992 describes a method of expressing an antibody in the mammary gland of a transgenic mammal. A transgene is constructed that includes a milk-specific promoter and nucleic acids encoding the antibody of interest and a signal sequence for secretion. The milk produced by females of such transgenic mammals includes, secreted-therein, the antibody of interest. The antibody can be purified from the milk, or for some applications, used directly.

**Pharmaceutical Compositions**

An antibody as described herein (e.g., DX-2930) can be present in a composition, e.g., a pharmaceutically acceptable composition or pharmaceutical composition. The antibody as described herein (e.g., DX-2930) can be formulated together with a pharmaceutically acceptable carrier. In some embodiments, 30 mg-400 mg of DX-2930 antibody are present in a composition, optionally with a pharmaceutically acceptable carrier, *e.g.,* a pharmaceutically acceptable composition or pharmaceutical composition. In some embodiments, 30 mg, 100 mg, 150 mg, 300 mg, or 400 mg of DX-2930 antibody are present in a composition optionally with a pharmaceutically acceptable carrier, *e.g.,* a pharmaceutically acceptable composition or pharmaceutical composition.

A pharmaceutically acceptable carrier includes any and all solvents, dispersion media, coatings, antibacterial and antifungal agents, isotonic and absorption delaying agents, and the like that are physiologically compatible. Preferably, the carrier is suitable for subcutaneous, intravenous, intramuscular, parenteral, spinal, or epidermal administration (e.g., by injection or infusion), although carriers suitable for inhalation and intranasal administration are also contemplated. In some embodiments, the pharmaceutically acceptable carrier is one or more of sodium phosphate, citric acid, histidine, sodium chloride, and Tween 80. In some embodiments, the pharmaceutically acceptable carrier is sodium phosphate, citric acid, histidine, sodium chloride, and Tween 80. In some embodiments, the antibody, such as DX-2930, is formulated in 30 mM sodium phosphate, 8.6 mM citric acid, 50 mM histidine, 90 mM sodium chloride, 0.01% Tween 80, pH 6.0. In some embodiments, the composition comprises or consists of 100 mg DX-2930 per 1 mL solution of 30 mM sodium phosphate, 8.6 mM citric acid, 50 mM histidine, 90 mM sodium chloride, 0.01% Tween 80.

A pharmaceutically acceptable salt is a salt that retains the desired biological activity of the compound and does not impart any undesired toxicological effects (see *e.g.,* Berge, S.M., et al., 1977, J. Pharm. Sci. 66:1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous, and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids, and the like. Base addition salts include those derived from alkaline earth metals, such as sodium, potassium, magnesium, calcium, and the like, as well as from nontoxic organic amines, such as N,N'-dibenzylethylenediamine, N-methylglucamine, chloroprocaine, choline, diethanolamine, ethylenediamine, procaine, and the like.

The compositions may be in a variety of forms. These include, for example, liquid, semi-solid and solid dosage forms, such as liquid solutions (e.g., injectable and infusible solutions), dispersions or suspensions, tablets, pills, powders, liposomes and suppositories. The form can depend on the intended mode of administration and therapeutic application. Many compositions are in the form of injectable or infusible solutions, such as compositions similar to those used for administration of humans with antibodies. An exemplary mode of administration is parenteral (e.g., intravenous, subcutaneous, intraperitoneal, intramuscular). In one embodiment, the plasma kallikrein binding protein is administered by intravenous infusion or injection. In another preferred embodiment, the plasma kallikrein binding protein is administered by intramuscular or subcutaneous injection. In another preferred embodiment, the plasma kallikrein binding protein is administered by intraperitoneal injection.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

The composition can be formulated as a solution, microemulsion, dispersion, liposome, or other ordered structure suitable to high drug concentration. Sterile injectable solutions can be prepared by incorporating the binding protein in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

An antibody as described herein (e.g., DX-2930) can be administered by a variety of methods, including intravenous injection or infusion. For example, for some therapeutic applications, the antibody can be administered by intravenous infusion at a rate of less than 30, 20, 10, 5, or 1 mg/min to reach a dose of about 1 to 100 mg/m$^2$ or 7 to 25 mg/m$^2$. The route and/or mode of administration will vary depending upon the desired results. In certain embodiments, the active compound may be prepared with a carrier that will protect the compound against rapid release, such as a controlled release formulation, including implants, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are available. See, e.g., Sustained and Controlled Release Drug Delivery Systems, J.R. Robinson, ed., 1978, Marcel Dekker, Inc., New York.

Pharmaceutical compositions can be administered with medical devices. For example, in one embodiment, a pharmaceutical composition disclosed herein can be administered with a device, e.g., a needleless hypodermic injection device, a pump, or implant.

In certain embodiments, an antibody as described herein (e.g., DX-2930) can be formulated to ensure proper distribution *in vivo*. For example, the blood-brain barrier (BBB) excludes many highly hydrophilic compounds. To ensure that the therapeutic compounds disclosed herein cross the BBB (if desired), they can be formulated, for example, in liposomes. For methods of manufacturing liposomes, see, e.g., U.S. Pat. Nos. 4,522,811; 5,374,548; and 5,399,331. The liposomes may comprise one or more moieties that are selectively transported into specific cells or organs, thus enhance targeted drug delivery (see, *e.g.,* V.V. Ranade, 1989, J. Clin. Pharmacol. 29:685).

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms can be dictated by and directly dependent on (a) the unique characteristics of the active compound and the particular therapeutic effect to be achieved, and (b) the limitations inherent in the art of compounding such an active compound for the treatment of sensitivity in individuals.

An exemplary, non-limiting range for a therapeutically or prophylactically effective amount of an antibody as described herein (e.g., DX-2930) is 30 mg to 400 mg, or any integer in between, for example, 100-400 mg, 100-300 mg, or 300-400 mg. In some embodiments, the therapeutically or prophylactically effective amount is 30 mg, 100 mg, 150 mg, 200 mg, 250 mg, 300 mg, 350 mg, or 400 mg.

In some embodiments, the therapeutically or prophylactically effective amount of an antibody described herein (e.g., DX-2930) is 30 mg, 100 mg, 150 mg, 300 mg, or 400 mg. In some embodiments, the therapeutically or prophylactically effective amount is 150 mg. In some embodiments, the therapeutically or prophylactically effective amount is 300 mg. In some embodiments, the therapeutically or prophylactically effective amount is 400 mg.

In some embodiments, the therapeutically or prophylactically effective amount is administered at least two times, at least three times, at least four times, at least five times, at least six times, at least seven times, at least eight times, at least nine times, at least ten times, or more. In some embodiments, the therapeutically or prophylactically effective amount is administered every other week (i.e., every two weeks). In some embodiments, the therapeutically or prophylactically effective amount is 300 mg or 400 mg and the amount is administered every two weeks. In some embodiments, the therapeutically or prophylactically effective amount is 300 mg and this amount of the antibody is administered every two weeks. In some embodiments, the therapeutically or prophylactically effective amount is 400 mg and this amount of the antibody is administered every two weeks.

In some embodiments, treatment of any of the anti-pKal antibody such as DX-2930 involves a treatment regimen comprising at least a loading period and a maintenance period. In some embodiments, the therapeutically or prophylactically effective amount of the antibody for the loading period is 100 to 300 mg (*e.g.,* 150 mg or 300 mg) per each administration. During this period, the antibody may be administered every week (*e.g.,* every week for one, two or three weeks). In one example, the loading period is 2 weeks and the antibody is administered at day 0, day 7, and day 14.

Alternatively or in addition, the therapeutically or prophylactically effective amount for the maintenance period is about 100 to 300 mg (*e.g.,* 150 mg or 300 mg) per each administration. During this period, the antibody can be administered every other week (*i.e.,* every two weeks), every three weeks, or every four weeks (*e.g.,* every two weeks for ten weeks, resulting in delivery of 5 doses total). In one example, the maintenance period may last for 10 weeks and the antibody is administered at day 28, day 42, day 56, day 70, and day 84.

In some embodiments, the anti-pKal antibody such as DX-2930 is administered at 150 mg or 300 mg and the amount is first administered every week for a suitable period (*e.g.,* every week for one, two or three weeks) and subsequently administered every two to four weeks (e.g., every two, three or four weeks) for a suitable period.

In any of the methods described herein, the treatment regimen may further comprise a follow-up period after the maintenance period. In the follow-up period, the antibody such as DX-2930 may be administered every 2-4 weeks at 100-300 mg, for example 300 mg. In some instances, the dosage may increase to 400 mg in one or more of the loading period, the maintenance period, and the follow-up period.

The pharmaceutical compositions disclosed herein may include a "therapeutically effective amount" or a "prophylactically effective amount" of an antibody as described herein (e.g., DX-2930).

### Kits

An antibody as described herein (e.g., DX-2930) can be provided in a kit, e.g., as a component of a kit. For example, the kit includes (a) a DX-2930 antibody, e.g., a composition (e.g., a pharmaceutical composition) that includes the antibody, and, optionally (b) informational material. The informational material can be descriptive, instructional, marketing or other material that relates to a method described herein and/or the use of an antibody as described herein (*e.g.,* DX-2930), *e.g.,* for a method described herein. In some embodiments, the kit comprises one or more doses of DX-2930. In some embodiments, the one or more doses are 30 mg, 100 mg, 150 mg, 300 mg or 400 mg.

The informational material of the kit is not limited in its form. In one embodiment, the informational material can include information about production of the compound, molecular weight of the compound, concentration, date of expiration, batch or production site information, and so forth. In one embodiment, the informational material relates to using the antibody to treat, prevent, or diagnosis of disorders and conditions, e.g., a plasma kallikrein associated disease or condition.

In one embodiment, the informational material can include instructions to administer n antibody as described herein (*e.g.*, DX-2930) in a suitable manner to perform the methods described herein, *e.g.,* in a suitable dose, dosage form, mode of administration or dosing schedule (*e.g.,* a dose, dosage form, dosing schedule or mode of administration described herein). In another embodiment, the informational material can include instructions to administer an antibody as described herein (*e.g.,* DX-2930) to a suitable subject, *e.g.,* a human, *e.g.,* a human having, or at risk for, a plasma kallikrein associated disease or condition. For example, the material can include instructions to administer an antibody as described herein (e.g., DX-2930) to a patient with a disorder or condition described herein, e.g., a plasma kallikrein associated disease, e.g., according to a dosing schedule described herein. The informational material of the kits is not limited in its form. In many cases, the informational material, e.g., instructions, is provided in print but may also be in other formats, such as computer readable material.

An antibody as described herein (e.g., DX-2930) can be provided in any form, e.g., liquid, dried or lyophilized form. It is preferred that an antibody be substantially pure and/or sterile. When an antibody is provided in a liquid solution, the liquid solution preferably is an aqueous solution, with a sterile aqueous solution being preferred. When an antibody is provided as a dried form, reconstitution generally is by the addition of a suitable solvent. The solvent, e.g., sterile water or buffer, can optionally be provided in the kit.

The kit can include one or more containers for the composition containing an antibody as described herein (e.g., DX-2930). In some embodiments, the kit contains separate containers, dividers or compartments for the composition and informational material. For example, the composition can be contained in a bottle, vial, or syringe, and the informational material can be contained in association with the container. In other embodiments, the separate elements of the kit are contained within a single, undivided container. For example, the composition is contained in a bottle, vial or syringe that has attached thereto the informational material in the form of a label. In some embodiments, the kit includes a plurality (e.g., a pack) of individual containers, each containing one or more unit dosage forms (e.g., a dosage form described herein) of an antibody as described herein (e.g., DX-2930). For example, the kit includes a plurality of syringes, ampules, foil packets, or blister packs, each containing a single unit dose of an antibody as described herein (e.g., DX-2930). The containers of the kits can be air tight, waterproof (e.g., impermeable to changes in moisture or evaporation), and/or light-tight.

The kit optionally includes a device suitable for administration of the composition, e.g., a syringe, or any such delivery device. In one embodiment, the device is an implantable device that dispenses metered doses of the antibody. The disclosure also features a method of providing a kit, e.g., by combining components described herein.

## Treatment

In some aspects, the disclosure provides the use of an antibody as described herein (e.g., DX-2930) in treating HAE.

*Hereditary Angioedema*

Hereditary angioedema (HAE) is also known as "Quincke edema," C1 esterase inhibitor deficiency, C1 inhibitor deficiency, and hereditary angioneurotic edema (HANE). HAE is characterized by recurrent episodes of severe swelling (angioedema), which can affect, e.g., the limbs, face, genitals, gastrointestinal tract, and airway. Symptoms of HAE include, e.g., swelling in the arms, legs, lips, eyes, tongue, and/or throat; airway blockage that can involve throat swelling and sudden hoarseness; repeat episodes of abdominal cramping without obvious cause; and/or swelling of the intestines, which can be severe and can lead to abdominal cramping, vomiting, dehydration, diarrhea, pain, and/or shock. About one-third of individuals with this HAE develop a non-itchy rash called erythema marginatum during an attack.

Swelling of the airway can be life threatening and causes death in some patients. Mortality rates are estimated at 15-33%. HAE leads to about 15,000-30,000 emergency department visits per year.

Trauma or stress, e.g., dental procedures, sickness (e.g., viral illnesses such as colds and the flu), menstruation, and surgery can trigger an attack of angioedema. To prevent acute attacks of HAE, patients can attempt to avoid specific stimuli that have previously caused attacks. However, in many cases, an attack occurs without a known trigger. Typically, HAE symptoms first appear in childhood and worsen during puberty. On average, untreated individuals have an attack every 1 to 2 weeks, and most episodes last for about 3 to 4 days (ghr.nlm.nih.gov/condition/hereditary-angioedema). The frequency and duration of attacks vary greatly among people with hereditary angioedema, even among people in the same family.

There are three types of HAE, known as types I, II, and III, all of which can be treated by the methods described herein. It is estimated that HAE affects 1 in 50,000 people, that type I accounts for about 85 percent of cases, type II accounts for about 15 percent of cases, and type III is very rare. Patients having type I or type II HAE are typically deficient in C1-INH. Such patients either have a defective C1-INH gene and thus do not produce C1-INH, or produce atypical C1-INH proteins. Type III is the most newly described form and was originally thought to occur only in women, but families with affected males have been identified. Type III HAE is believed to be unassociated with C1-INH. Patients having type III HAE may have normal C1-INH proteins.

HAE is inherited in an autosomal dominant pattern, such that an affected person can inherit the mutation from one affected parent. New mutations in the gene can also occur, and thus HAE can also occur in people with no history of the disorder in their family. It is estimated that 20-25% of cases result from a new spontaneous mutation.

Mutations in the SERPING1 gene cause hereditary angioedema type I and type II. The SERPING1 gene provides instructions for making the C1 inhibitor protein, which is important for controlling inflammation. C1 inhibitor blocks the activity of certain proteins that promote inflammation. Mutations that cause hereditary angioedema type I lead to reduced levels of C1 inhibitor in the blood. In contrast, mutations that cause type II result in the production of a C1 inhibitor that functions abnormally. Without the proper levels of functional C1 inhibitor, excessive amounts of bradykinin are generated. Bradykinin promotes inflammation by increasing the leakage of fluid through the walls of blood vessels into body tissues. Excessive accumulation of fluids in body tissues causes the episodes of swelling seen in individuals with hereditary angioedema type I and type II.

Mutations in the F12 gene are associated with some cases of hereditary angioedema type III. The F12 gene provides instructions for making coagulation factor XII. In addition to playing a critical role in blood clotting (coagulation), factor XII is also an important stimulator of inflammation and is involved in the production of bradykinin. Certain mutations in the F12 gene result in the production of factor XII with increased activity. As a result, more bradykinin is generated and blood vessel walls become more leaky, which leads to episodes of swelling. The cause of other cases of hereditary angioedema type III remains unknown. Mutations in one or more as-yet unidentified genes may be responsible for the disorder in these cases.

HAE can present similarly to other forms of angioedema resulting from allergies or other medical conditions, but it differs significantly in cause and treatment. When hereditary angioedema is misdiagnosed as an allergy, it is most commonly treated with antihistamines, steroids, and/or epinephrine, which are typically ineffective in HAE, although epinephrine can be used for life-threatening reactions. Misdiagnoses have also resulted in unnecessary exploratory surgery for patients with abdominal swelling, and in some HAE patients abdominal pain has been incorrectly diagnosed as psychosomatic.

C1 inhibitor therapies, as well as other therapies for HAE, are described in Kaplan, A.P., J Allergy Clin Immunol, 2010, 126(5):918-925.

Acute treatment of HAE attacks is provided to halt progression of the edema as quickly as possible. C1 inhibitor concentrate from donor blood, which is administered intravenously, is one acute treatment; however, this treatment is not available in many countries. In emergency situations where C1 inhibitor concentrate is not available, fresh frozen plasma (FFP) can be used as an alternative, as it also contains C1 inhibitor.

Purified C1 inhibitor, derived from human blood, has been used in Europe since 1979. Several C1 inhibitor treatments are now available in the U.S. and two C1 inhibitor products are now available in Canada. Berinert P (CSL Behring), which is pasteurized, was approved by the F.D.A. in 2009 for acute attacks. Cinryze (ViroPharma), which is nanofiltered, was approved by the F.D.A. in 2008 for prophylaxis. Rhucin (Pharming) is a recombinant C1 inhibitor under development that does not carry the risk of infectious disease transmission due to human blood-borne pathogens.

Treatment of an acute HAE attack also can include medications for pain relief and/or IV fluids.

Other treatment modalities can stimulate the synthesis of C1 inhibitor, or reduce C1 inhibitor consumption. Androgen medications, such as danazol, can reduce the frequency and severity of attacks by stimulating production of C1 inhibitor.

Helicobacter pylori can trigger abdominal attacks. Antibiotics to treat h. pylori will decrease abdominal attacks.

Newer treatments attack the contact cascade. Ecallantide (KALBITOR®, DX-88, Dyax) inhibits plasma kallikrein and has been approved in the U.S.. Icatibant (FIRAZYR®, Shire) inhibits the bradykinin B2 receptor, and has been approved in Europe and the U.S.

Diagnosis of HAE can rely on, e.g., family history and/or blood tests. Laboratory findings associated with HAE types I, II, and III are described, e.g., in Kaplan, A.P., J Allergy Clin Immunol, 2010, 126(5):918-925. In type I HAE, the level of C1 inhibitor is decreased, as is the level of C4, whereas C1q level is normal. In type II HAE, the level of C1 inhibitor is normal or increased; however, C1 inhibitor function is abnormal. C4 level is decreased and C1q level is normal. In type III, the levels of C1 inhibitor, C4, and C1q can all be normal.

Symptoms of HAE can be assessed, for example, using questionnaires, e.g., questionnaires that are completed by patients, clinicians, or family members. Such questionnaires are known in the art and include, for example, visual analog scales. See, *e.g.,* McMillan, C.V. et al. Patient. 2012;5(2):113-26.

*Treating HAE with anti-PKal antibodies*

The disclosure provides methods of treating (*e.g.*, ameliorating, stabilizing, or eliminating one or more symptoms) of hereditary angioedema (HAE) by administering an antibody described herein (*e.g.,* a therapeutically effective amount of an antibody described herein) to a subject having or suspected of having HAE, *e.g.,* according to a dosing schedule described herein. Additionally provided are methods of treating HAE by administering an antibody described herein (*e.g.,* a therapeutically effective amount of an antibody described herein), e.g., according to a dosing schedule described herein, or in combination with a second therapy, e.g., with one other agent, e.g., described herein. The disclosure also provides methods of preventing HAE or a symptom thereof by administering an antibody described herein (*e.g., a* prophylactically effective amount of an antibody described herein) to a subject at risk of developing HAE (*e.g., a* subject having a family member with HAE or a genetic predisposition thereto), *e.g.,* according to a dosing schedule described herein. In some examples, the subject may be a human patient who has no HAE symptoms at the time of the treatment.

Treating includes administering an amount effective to alleviate, relieve, alter, remedy, ameliorate, improve or affect the disorder, the symptoms of the disorder or the predisposition toward the disorder. The treatment may also delay onset, e.g., prevent onset, or prevent deterioration of a disease or condition.

Methods of administering DX-2930 antibodies are also described in "Pharmaceutical Compositions." Suitable dosages of the antibody used can depend on the age and weight of the subject and the particular drug used. The antibody can be used as competitive agents to inhibit, reduce an undesirable interaction, e.g., between plasma kallikrein and its substrate (e.g., Factor XII or HMWK). The dose of the antibody can be the amount sufficient to block 90%, 95%, 99%, or 99.9% of the activity of plasma kallikrein in the patient, especially at the site of disease. This may require 30 mg, 100 mg, 300 mg, or 400 mg, e.g., administered every two weeks.

In one embodiment, the antibodies are used to inhibit an activity (e.g., inhibit at least one activity of plasma kallikrein, e.g., reduce Factor XIIa and/or bradykinin production) of plasma kallikrein, e.g., *in vivo.* The binding proteins can be used by themselves or conjugated to an agent, e.g., a cytotoxic drug, cytotoxin enzyme, or radioisotope.

The antibodies can be used directly *in vivo* to eliminate antigen-expressing cells via natural complement-dependent cytotoxicity (CDC) or antibody dependent cellular cytotoxicity (ADCC). The antibodies described herein can include complement binding effector domain, such as the Fc portions from IgG1, -2, or -3 or corresponding portions of IgM which bind complement. In one embodiment, a population of target cells is ex vivo treated with an antibody described herein and appropriate effector cells. The treatment can be supplemented by the addition of complement or serum containing complement. Further, phagocytosis of target cells coated with an antibody described herein can be improved by binding of complement proteins. In another embodiment target, cells coated with the antibody which includes a complement binding effector domain are lysed by complement.

Methods of administering DX-2930 antibodies are described in "Pharmaceutical Compositions." Suitable dosages of the molecules used will depend on the age and weight of the subject and the particular drug used. The antibodies can be used as competitive agents to inhibit or reduce an undesirable interaction, e.g., between a natural or pathological agent and the plasma kallikrein.

A therapeutically effective amount of an antibody as described herein, can be administered to a subject having, suspected of having, or at risk for HAE, thereby treating (e.g., ameliorating or improving a symptom or feature of a disorder, slowing, stabilizing and/or halting disease progression) the disorder.

The antibody described herein can be administered in a therapeutically effective amount. A therapeutically effective amount of an antibody is the amount which is effective, upon single or multiple dose administration to a subject, in treating a subject, e.g., curing, alleviating, relieving or improving at least one symptom of a disorder in a subject to a degree beyond that expected in the absence of such treatment.

Dosage regimens can be adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

In some embodiments, the DX-2930 antibody is administered by multiple doses such as once every 2 weeks, once every 3 weeks, once every four weeks, once every 6 weeks, once every 8 weeks or less frequent. Each of the multiple doses can be 30 mg, 100 mg, 150 mg, 300 mg, 350 mg. or 400 mg. In some instances, a patient may be given multiple doses once every 2 weeks, for a suitable period of time. In some embodiments, DX-2930 can be administered at 300 mg or 400 mg every two weeks. In other embodiments, DX-2930 can be administered at 300 mg or 400 mg every four weeks. In yet other embodiments, DX-2930 can be administered at 150 mg every four weeks. In any of the methods described herein, a subject treated by DX-2930 for multiple doses as described herein may be followed up with a maintenance treatment.

In any of the methods described herein, a subject may be treated by DX-2930 for multiple doses in a loading period and then followed up with a maintenance period. In the loading period, the subject may be treated with DX-2930 at about 100 mg to about 400 mg (e.g., 100-300 mg or 150-300 mg, for example, 100 mg, 150 mg, 200 mg, 300 mg, or 400 mg) once every 2-4 weeks (for example, every 2 weeks, every 3 weeks, or every 4 weeks) for a suitable period. In the loading period, the subject may be treated with DX-2930 at about 100 mg to about 300 mg (e.g., 100-300 mg or 150-300 mg, for example, 100 mg, 150 mg, 200 mg, or 300 mg) once every 2-4 weeks (for example, every 2 weeks, every 3 weeks, or every 4 weeks) for a suitable period.

In some embodiments, the patient can be monitored for side effects (e.g., elevation of creatine phosphatase levels) and/or inhibition levels of pKal by the antibody (e.g., serum or plasma concentration of the antibody or the pKal activity level) before and after the treatment or during the course of treatment. If adverse effect is observed, the dose of the antibody might be reduced or the treatment might be terminated. If the inhibition level is below a minimum therapeutic level, further doses of the antibody might be administered to the patient.

In some embodiments, the plasma or serum concentration of the antibody (e.g., DX-2930) may be measured during the course of the treatment (e.g., after the initial dosage) for assessing the efficacy of the treatment. If the plasma or serum concentration of the antibody is lower than about 80 nM, a follow-up dosage may be needed, which may be the same or higher than the initial dosage. The plasma or serum concentration of the antibody may be measured by determining the protein level of the antibody in a plasma or serum sample obtained from the subject, e.g., by an immune assay or MS assay. The plasma or serum concentration of the antibody may also be measured by determining the inhibitory level of pKal in a plasma or serum sample obtained from a subject treated with the antibody. Such assays may include the synthetic substrate assay or the Western blot assay for measuring cleaved kininogen as described herein.

Alternatively or in addition, the plasma or serum level of creatine kinase can be monitored during the course of the treatment. If the plasma or serum level of creatine kinase is found to elevate during the treatment, the dosage of the antibody may be reduced or the treatment may be terminated.

In some embodiments, an optimal dosage (e.g., optimal prophylactic dosage or optimal therapeutic dosage) of the antibody (e.g., DX-2930) may be determined as follows. The antibody is given to a subject in need of the treatment at an initial dose. The plasma concentration of the antibody in the subject is measured. If the plasma concentration is lower than 80 nM, the dose of the antibody is increased in a subsequent administration. A dosage of the antibody that maintains the antibody plasma concentration above about 80 nM can be chosen as the optimal dosage for the subject. The creatine phosphokinase level of the subject can be monitored during the course of treatment and the optimal dosage for that subject can be further adjusted based on the creatine phosphokinase level, e.g., the dosage of the antibody might be reduced is elevation of creatine phosphokinase is observed during treatment. In some embodiments, the antibody such as DX-2930 is administered to reduce the level of cleaved kininogen to levels comparable to healthy subjects.

In some embodiments, any of the antibodies disclosed herein, such as DX-2930 and its functional variants, may be used to prevent HAE attack or reduce the rate of HAE attack in human patients having history of HAE attack. In some examples, the human patients experienced at least two HAE attacks per year and optionally at least one within the 6 months prior to the treatment. In other examples, the human patients experienced at least two HAE attacks within 3 months prior to the treatment. In other examples, the human patients had at least 9 HAE attacks within 3 months prior to the treatment and optionally at least 25 attacks (*e.g.,* 36 attacks) within 12 months prior to the treatment.

## Combination Therapies

An antibody as described herein (e.g., DX-2930) can be administered in combination with one or more of the other therapies for treating a disease or condition associated with plasma kallikrein activity, *e.g.,* a disease or condition described herein. For example, an antibody as described herein (e.g., DX-2930) can be used therapeutically or prophylactically with surgery, another anti- plasma kallikrein Fab or IgG (*e.g.,* another Fab or IgG described herein), another plasma kallikrein inhibitor, a peptide inhibitor, or small molecule inhibitor. Examples of plasma kallikrein inhibitors that can be used in combination therapy with a plasma kallikrein binding antibodies described herein include plasma kallikrein inhibitors described in, *e.g.,* WO 95/21601 or WO 2003/103475.

One or more plasma kallikrein inhibitors can be used in combination with an antibody as described herein (e.g., DX-2930). For example, the combination can result in a lower dose of the inhibitor being needed, such that side effects are reduced.

An antibody as described herein (e.g., DX-2930) can be administered in combination with one or more current therapies for treating HAE. For example, DX-2930 antibody can be co-used with a second anti-HAE therapeutic agent such as ecallantide, a C1 esterase inhibitor (e.g., CINRYZE™), aprotinin (TRASYLOL®), and/or a bradykinin B2 receptor inhibitor (*e.g*., icatibant (FIRAZYR®)).

The term "combination" refers to the use of the two or more agents or therapies to treat the same patient, wherein the use or action of the agents or therapies overlaps in time. The agents or therapies can be administered at the same time (*e.g.,* as a single formulation that is administered to a patient or as two separate formulations administered concurrently) or sequentially in any order. Sequential administrations are administrations that are given at different times. The time between administration of the one agent and another agent can be minutes, hours, days, or weeks. The use of a plasma kallikrein binding antibody described herein can also be used to reduce the dosage of another therapy, *e.g.,* to reduce the side effects associated with another agent that is being administered. Accordingly, a combination can include administering a second agent at a dosage at least 10, 20, 30, or 50% lower than would be used in the absence of the plasma kallikrein binding antibody.

A combination therapy can include administering an agent that reduces the side effects of other therapies. The agent can be an agent that reduces the side effects of a plasma kallikrein associated disease treatment.

Without further elaboration, it is believed that one skilled in the art can, based on the above description, utilize the present invention to its fullest extent. The following specific embodiments are, therefore, to be construed as merely illustrative, and not limitative of the remainder of the disclosure in any way whatsoever. All publications cited herein are incorporated by reference for the purposes or subject matter referenced herein.

## EXAMPLES

### Example 1: A Phase 1b, Double-Blind, Multiple Ascending Dose Study to Assess Safety, Tolerability and Pharmacokinetics of DX-2930 in Hereditary Angioedema Subjects

A phase 1b trial was conducted to assess safety and tolerability of multiple subcutaneous administrations of DX-2930 at different dose levels in hereditary angioedema (HAE) subjects. HAE patients included in the study included those with documented diagnosis of HAE (Type I or Type II) based upon all of the following: documented clinical history consistent with HAE (subcutaneous or mucosal, nonpruritic swelling episodes without accompanying urticaria), C1 inhibitor (C1-INH) antigen or functional level < 40% of the normal level (Subjects with antigen or functional C1-INH level 40-50% of the normal level were enrolled if they also had a C4 level below the normal range and a family history consistent with HAE Type I or II), age at reported onset of first angioedema symptoms $\leq$ 30 years or a family history consistent with HAE Type I or II, and experiencing $\geq$2 HAE attacks per year, with at least 1 attack in the past 6 months reported by the subject. HAE patients enrolled were randomized in a 2:1 ratio of active drug to placebo and administered subcutaneous doses of DX-2930 at 30 mg (n=4), 100 mg (n=4), 300 mg (n=5), and 400 mg (n=11) or placebo (n=13) in two doses separated by 14 days. One patient in the 400 mg group received only one dose and then was unavailable for the second dose and was replaced. The replacement patient was also unable to complete the study for reasons not related to the study, resulting a total of 10 patients completing the 400 mg dose and being included in the assessment. Plasma was collected at time points following administration out to day 120 (15 weeks), except in the group that received the 400 mg dose, where data was available up to day 50. Analyses included safety, pharmacokinetics, pharmacodynamics (biomarkers), and efficacy. The drug group and the placebo group were baled in terms of age, race, ethnicity, and BMI, although there were slightly more females present in the DX-2930 (67%) vs placebo (54%) groups.

Pharmacokinetics of DX-2930

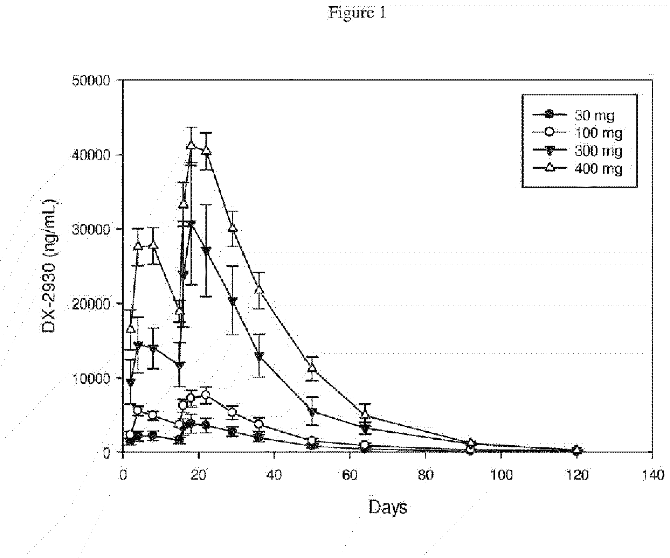

DX-2930 drug levels were measured in plasma by a validated immunoassay that used an anti-idiotypic antibody against DX-2930 (M293-D02). From the plot of mean plasma drug levels for each dose group versus days following DX-2930 administration, it was evident that drug levels were dose dependent and exhibited a prolonged half-life, typical of a human monoclonal antibody. Key pharmacokinetic observations are summarized in Table 2 and Figure 1. First, $C_{max}$ drug levels increased with increasing dose, as expected. In addition, the $T_{max}$ following the second dose was about 20 days and the half-life was approximately 14 days. These parameters were consistent with values obtained in a phase 1a study in healthy volunteers and supported either once or twice a month dosing.

**Table 2. Pharmacokinetic observations**

| Doses | $C_{max}$ (ng/ml) | $T_{max}$ (days) | $t^{\frac{1}{2}}$ (days) |
|---|---|---|---|
| 30 mg | 3895.0 (2159.8) | 17.9 (1.1) | 14.2 (0.8) |
| 100 mg | 7890.0(2058.4) | 18.0 (0.5) | 14.6 (3.4) |
| 300 mg | 27460.0 (14542.5) | 18.2 (1.5) | 13.8 (3.3) |
| 400 mg | 45322.2 (8704.6) | 17.7 (1.0) | 15.0 (2.4) |

Values presented are the mean values for the evaluated patients with the standard deviation in parentheses.

Pharmacodynamic Activity of DX-2930: Fluorogenic Activity Assay

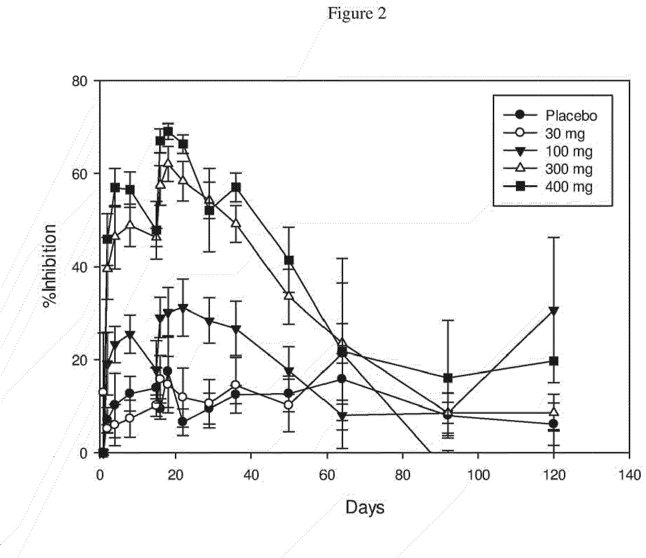

Two different biomarker assays were used to investigate the pharmacodynamic (PD) activity of DX-2930 in HAE patient plasma. The first PD assay is referred to as the Fluorogenic Activity assay, and it provides a measure of the bioactivity of DX-2930 in citrated plasma obtained from treated patients at the same time points following dosing as used to determine the pharmacokinetic properties of DX-2930. This assay measured the amount of active plasma kallikrein that is generated in diluted plasma after activation of the contact pathway. Specifically, dilute plasma was spiked with active Factor XIIa (FXIIa) which propagates the enzymatic cascade of the contact pathway, after 2 minutes the FXIIa inhibitor Corn Trypsin Inhibitor was added to stop the reaction, and the amount of active plasma kallikrein present in the sample was measured by its ability to hydrolyze a pro-fluorescent synthetic peptide substrate. The presence of increasing levels of DX-2930 in the plasma of treated healthy volunteers (phase 1a study) or HAE patients (phase 1b study) was associated with a dose dependent reduction in the observed plasma kallikrein enzymatic rate (Figure 2). The percent inhibition observed at each time point was calculated relative to the amount of plasma kallikrein activity in the pre-dose samples for each patient. The observed bioactivity in Figure 2 was well correlated with the pharmacokinetic properties of DX-2930 in Figure 1. Marked inhibition was observed in the 300 and 400 mg doses following dosing. Intermediate inhibition was observed in the 100 mg dose and no apparent inhibition was evident in the 30 mg dose.

Pharmacodynamic Activity of DX-2930: Western Blot Assay

HAE patients are deficient in C1-Inhibitor, the endogenous inhibitor of plasma kallikrein. As a result, these patients have elevated active plasma kallikrein, which converts its 1-chain high molecular weight kininogen substrate to 2-chain and bradykinin, the key mediator of pain and edema in HAE. DX-2930 is a highly potent inhibitor of active plasma kallikrein that blocks 2-chain and bradykinin generation.

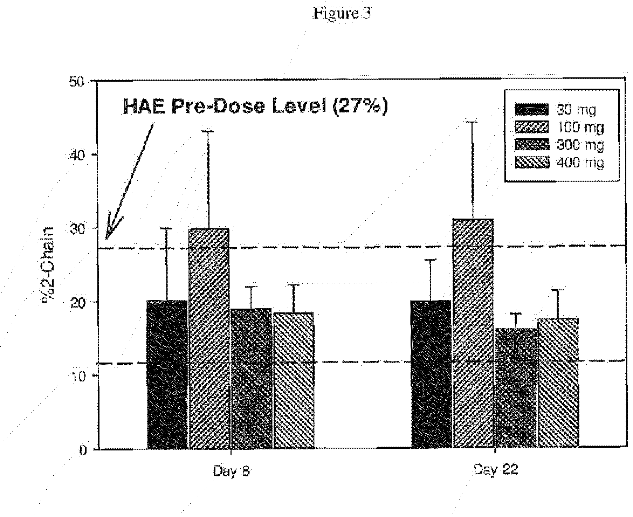

A Western blot assay was developed to measure the relative amounts of 1-chain and 2-chain high molecular weight kininogen in plasma in different plasma anticoagulants and treatment conditions. Figure 3 shows the %2-chain that was observed in HAE patient plasma collected in the presence of protease inhibitors (SCAT169 plasma). The presence of SCAT169 plasma prevented the activation of the contact system and subsequent 2-chain generation that can occur during blood collection and processing to plasma. Hence, this level of 2-chain was expected to closely match that of endogenous levels in HAE patients in in the phase 1b trial (27%) and in healthy volunteers (12%). DX-2930 treated HAE patients exhibited lower 2-chain levels as measured in samples collected either 8 or 22 days after dosing.

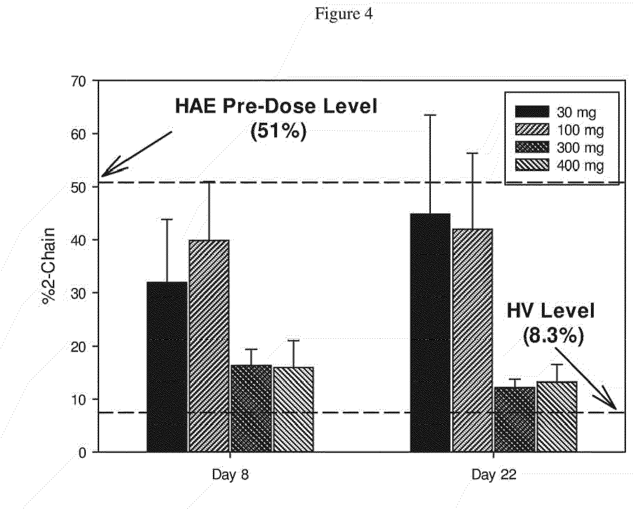

In Figure 4, pre-dose citrated plasma obtained from the HAE patients in the phase 1b trial contained approximately 52% 2-chain. In contrast, citrated plasma samples from healthy volunteers obtained in a phase 1a study contained approximately 8% 2-chain. Mean 2-chain levels in plasma of phase 1b subjects collected on days 8 and 22 were also investigated and are shown in Figure 4. The statistically significant reduction in 2-chain levels in the 300 and 400 mg dose groups versus pre-dose levels demonstrated pharmacodynamic activity of DX-2930.

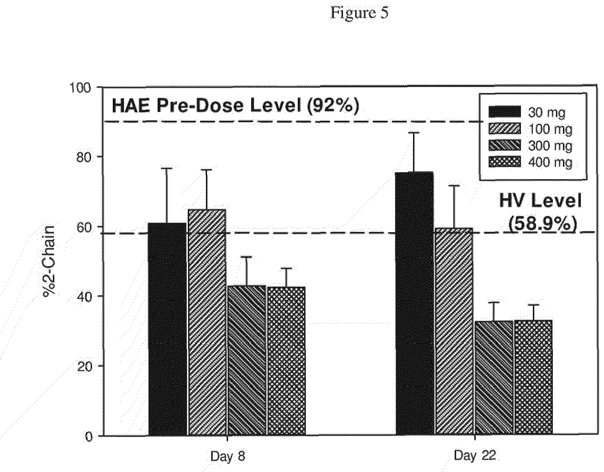

Figure 5 shows that citrated plasma from DX-2930 dosed HAE patients exhibited less 2-chain following ex vivo activation with coagulation factor XIIa (FXIIa). The 300 and 400 mg dose groups reduced the amount of 2-chain to a level below that observed in healthy volunteers. This *ex vivo* activation may be considered an *in vitro* model of a severe HAE attack.

Both of these PD biomarker assays supported dose selection that would achieve drug levels obtained at the 300 and 400 mg dose groups.

Safety

A summary of adverse events is shown in Table 3 below. There was no imbalance in treatment emergent adverse events (TEAEs) that would indicated a safety concern of DX-2930. Most common AEs were HAE attacks, injection site pain, and headache. There were 3 severe TEAEs (injection site pain lasting 1 min, worsening headache lasting 1 minute and night sweats). No safety signals were identified for clinical laboratory abnormalities or changes from baseline, vital signs or physical examinations, or abnormalities or changes in electrocardiogram (ECG). These results suggest that DX-2930 appears to be well tolerated in HAE patients at doses up to 400 mg.

**Table 3. Summary of treatment adverse events**

| | DX-2930 30 mg (N = 4) | DX-2930 100 mg (N = 4) | DX-2930 300 mg (N = 5) | DX-2930 400 mg (N = 11) | All DX-2930 treated (N = 24) | Placebo (N = 13) |
|---|---|---|---|---|---|---|
| TEAEs* | 1 | 3 | 2 | 8 | 14 (58%) | 10 (77%) |
| Deaths or subject discontinuations | 0 | 0 | 0 | 0 | 0 | 0 |
| Serious adverse events | 0 | 0 | 0 | 0 | 0 | 1 (8%) |
| Severe TEAEs | 1 | 0 | 2 | 2 | 5 (21%) | 5 (39%) |
| Related TEAEs** | 1 | 2 | 0 | 4 | 7 (29%) | 5 (39%) |
| * TEAE: Treatment Emergent Adverse Events. An AE is treatment emergent if the onset time is after administration of study drug through the Day 120 post dose final follow-up visit, or in the event that onset time precedes study drug administration, the AE increases in severity during the 120 day post dose follow-up period. ** Treatment-related AEs: Relatedness of AEs to study drug was assessed by a blinded investigator. | | | | | | |

Immunogenicity

5 patients were anti-drug antibody positive (2 of the 5 had intermittent, fluctuating results). However, no positive samples were neutralizing, there was no clinical evidence of hypersensitivity, and no apparent effects on pharmacokinetics or biomarkers.

Efficacy assessment

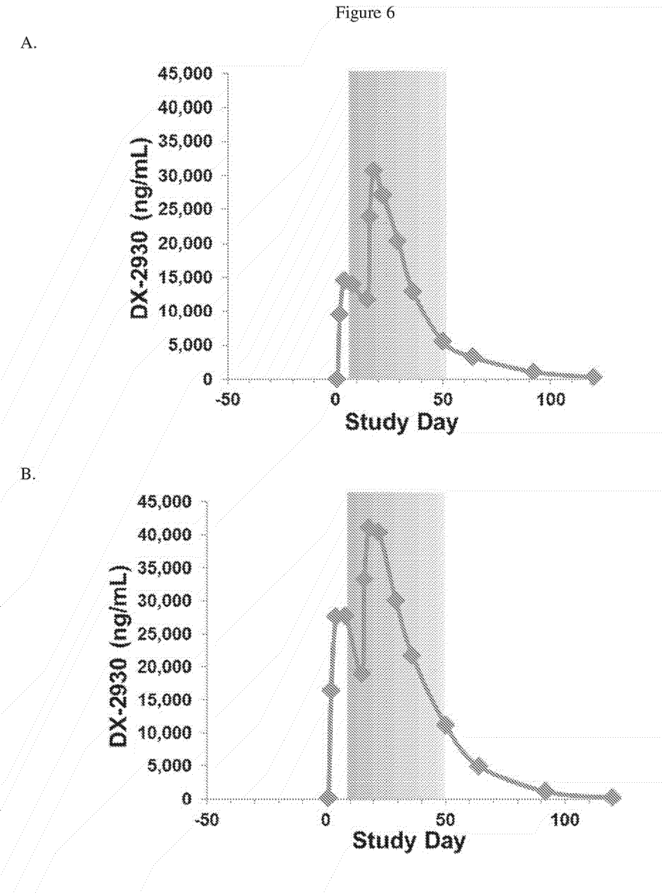

A prospective primary efficacy analysis was performed, which focused on the 300 and 400 mg doses of DX-2930, individually and combined, compared to placebo. A six week primary assessment period (Day 8 to Day 50) was used (Figure 6A and 6B), as PK modeling from phase 1a suggested notable drug exposure during this time interval. The primary analysis focused upon subjects with a minimum baseline history of at least 2 attacks in the past 3 months. Most of the subjects fulfilled the minimum required baseline history of at least 2 attacks in the past 3 months. Of the 13 placebo subjects, 11 met this requirement. Of the 16 subjects treated with 300 or 400 mg DX-2930, 15 met this requirement. The baseline HAE attack rates in the placebo, 300 mg, and 400 mg groups were 0.39, 0.33, and 0.55 attacks per week, respectively. The baseline rate in the combined 300 and 400 mg group was 0.49 attacks per week.

The primary approach was an intent-to-treat (ITT) analysis. A model of repeated measurements was used, with an analysis of variance (ANOVA) employing baseline attack rates as a covariate. The read-out was expressed as a percent reduction in HAE attacks by DX-2930 in comparison to the placebo attack rates, and p values were calculated.

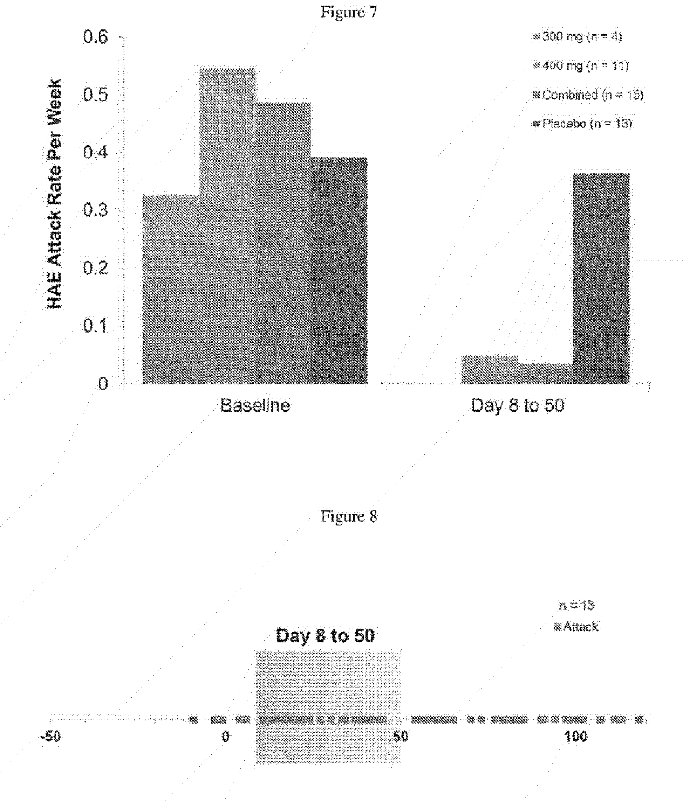

Results of the efficacy assessment are shown in Table 4 and 5 and in Figure 7, which both show a reduction in HAE attack rate with the 300 and 400 mg doses of DX-2930, individually and combined, compared to the placebo. In particular, 13 of the 15 DX-2930 subjects treated with 300 or 400 mg were attack-free for the duration of the study, whereas for the placebo group, only 3 of the 11 subjects were attack-free.

**Table 4. Reduction in HAE attack rate from day 8 to 50**

|  | DX-2930 300 mg (N = 4) | DX-2930 400 mg (N = 11) | DX-2930 Combined 300 and 400 mg (N = 15) |
|---|---|---|---|
| % Reduction vs placebo | 100 | 88 | 91 |
| P-value vs placebo* | <0.0001 | 0.005 | 0.0012 |
| Note: Only subjects who have a baseline attack rate of at least 2 attacks in the last 3 months were included. * Mixed Model Repeated Measurements with Analysis of Variance (baseline attack frequency as covariate) and assuming Poisson distribution. | | | |

**Table 5. Proportion of subjects who were attack-free**

|  | DX-2930 300 mg (N = 4) | DX-2930 400 mg (N = 11) | DX-2930 Combined 300 and 400 mg (N = 15) | Placebo (N = 11) |
|---|---|---|---|---|
| Attack-free subjects (Day 8 to 50) | 4/4 (100%) p = 0.026 | 9/11 (82%) p = 0.030 | 13/15 (87%) p = 0.004 | 3/11 (27%) |

Among the placebo subjects, there were a total of 24 attacks during the primary efficacy assessment period. Of these 24 attacks, the primary attack location was abdominal in 13 and laryngeal in 1 of them. 10 of the attacks were severe and 6 were moderate. Acute treatment for the attacks was received in 22 of the 24 attacks.

Among subjects treated with 300 or 400 mg DX-2930, one subject treated with 400 mg had a single HAE attack. This attack was peripheral, mild, lasted 8 hours, and did not require any acute treatment. The other 400 mg DX-2930-treated subject experiencing attacks had two peripheral attacks. One attack was moderate and the other was severe; both attacks were treated with acute therapy. A summary of the characteristics of the HAE attacks is shown in Table 6.

**Table 6. Characteristics of HAE attacks (Day 8 to 50)**

|  | DX-2930 300 mg (N = 4) | DX-2930 400 mg (N = 11) | Placebo (N = 11) |
|---|---|---|---|
| Attacks | 0 | 3 | 24 |
| **Primary Attack Location** |  |  |  |
| Peripheral | 0 | 3 | 10 |
| Abdominal | 0 | 0 | 13 |
| Laryngeal | 0 | 0 | 1 |
| **Attack Severity** |  |  |  |
| Mild | 0 | 1 | 8 |
| Moderate | 0 | 1 | 6 |
| Severe | 0 | 1 | 10 |
| Acute attacks requiring treatment | 0 | 2 | 22 |

Next, a modified intent-to-treat (mITT) post-hoc analysis was undertaken. The ITT population was used except 2 subjects were excluded (one subject that did not receive 2 administrations and 1 subject that did not have HAE type 1 or type 2). Results of the modified analysis are shown in Table 7. In this modified intent-to-treat analysis, from Day 8 to Day 50 in comparison to placebo, the 300 mg DX-2930 group had a 100% reduction in attacks with a p value of less than 0.0001. The 400 mg DX-2930 group had a 95% reduction in attacks with a p value of 0.0022. The combined 300 and 400 mg DX-2930 group had a 97% reduction in HAE attacks, with a p value of 0.0007.

**Table 7. Primary efficacy analysis (Day 8 to 50) (modified intent-to-treat analysis)**

| | DX-2930 300 mg (N = 4) | DX-2930 400 mg (N = 9) | DX-2930 Combined 300 and 400 mg (N = 13) |
|---|---|---|---|
| % Reduction vs placebo | 100 | 95 | 97 |
| P-value vs placebo* | <0.0001 | 0.0022 | 0.0007 |
| Note: Only subjects who have a baseline attack rate of at least 2 attacks in the last 3 months are included. MITT population excludes 2 subjects, one subject without type I or II HAE and one subject who received only 1 administration of DX-2930. <br> * Mixed Model Repeated Measurements with Analysis of Variance (baseline attack frequency as covariate) and assuming Poisson distribution. | | | |

The incidence of HAE attacks in relation to drug exposure over time was also evaluated. Without wishing to be bound by theory, the hypothesis was that higher drug levels should be correlated with prevention of HAE attacks, meaning (a) that before dosing as well as in the few days after dosing, attacks should be observed, (b) that as drug levels accumulate, attacks should become rare or even absent, and (c) that as the drug levels decline, attacks should re-emerge. The results of this evaluation are shown in Figures 8-13.

In the placebo group high incidence of attacks was evident (Figure 8). These events were distributed throughout the entire duration of the study without any particular pattern.

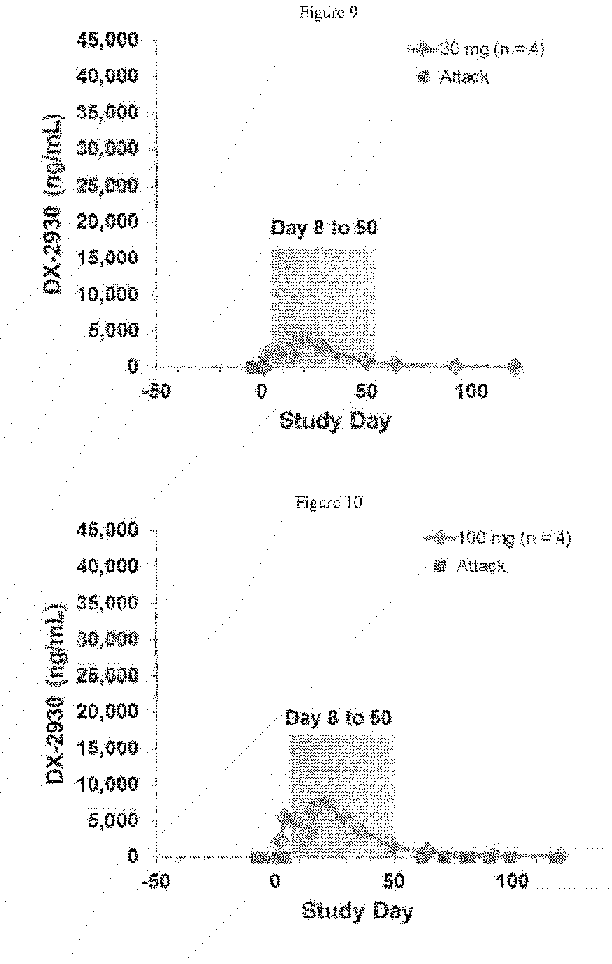

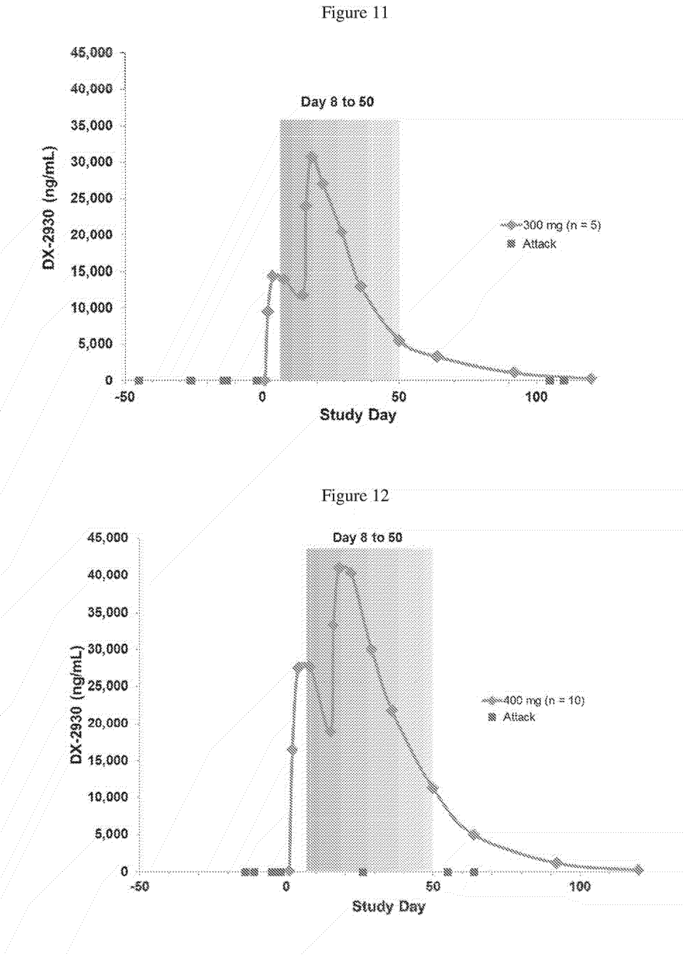

In the 30 and 100 mg DX-2930 groups, no attacks occurred from Day 8 to Day 50 (Figures 9 and 10). However, the baseline attack rates for these groups were relatively lower and the pharmacodynamic effect for the 30 mg group was not appreciably different from that for placebo. In the 300 mg DX-2930 group, there were attacks that occurred prior to dosing. As drug levels rose, the subjects became attack-free and as drug levels declined, attacks re-emerged (Figure 11). A similar pattern was also observed in the 400 mg DX-2930 group (Figure 12). Attack incidence substantially decreased during a period of notable drug exposure, particularly within the Day 8 to Day 50 time interval. This period was bracketed by attacks occurring during times of lower drug exposure, either prior to drug accumulation or as drug levels waned. These results show that there is a clear association between DX-2930 drug exposure and prevention of HAE attacks.

To further assess the efficacy of DX-2930, the therapeutic effects of DX-2930 were also observed in HAE patients with high baseline attack rates were explored. Subjects with at least 9 attacks in the past 3 months prior to dosing were identified and evaluated. There were 6 such subjects- 1 treated with placebo, 1 treated with 300 mg DX-2930, and 4 treated with 400 mg DX-2930.

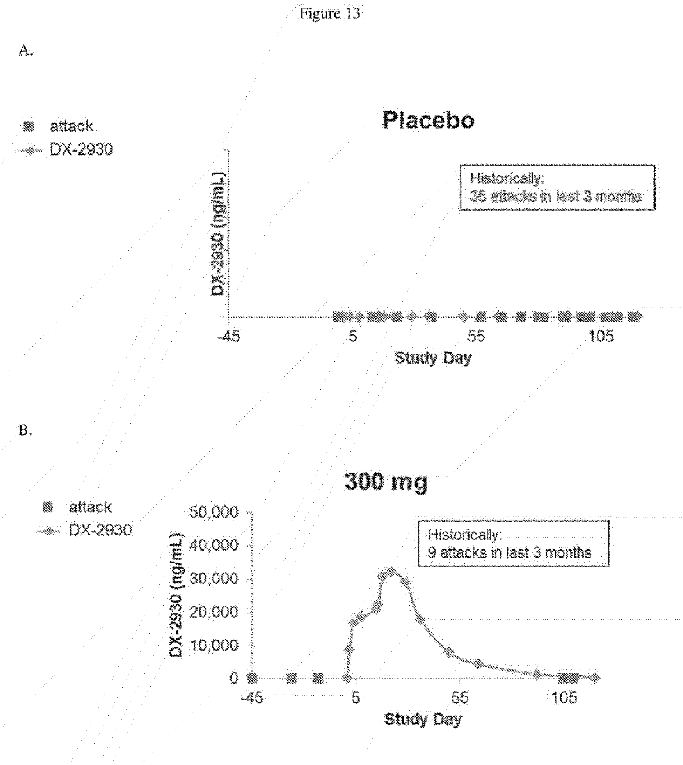

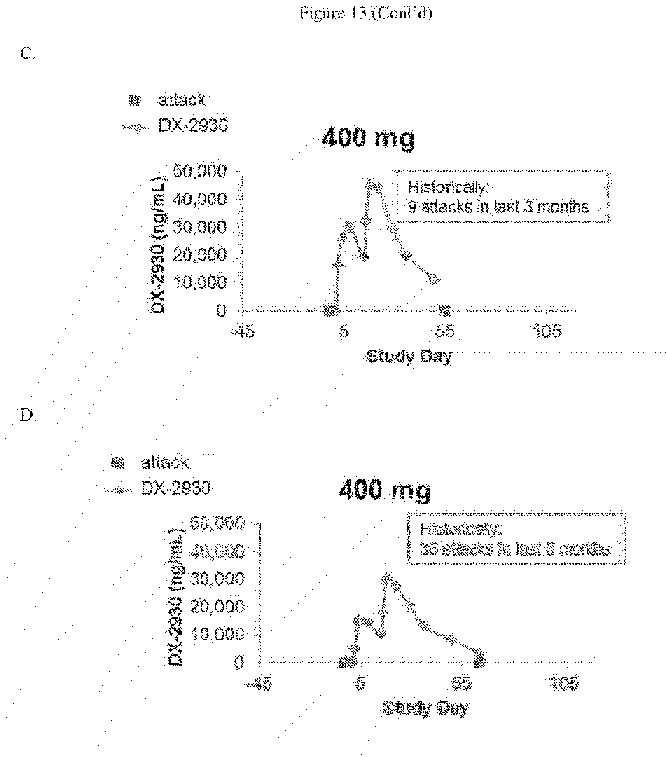

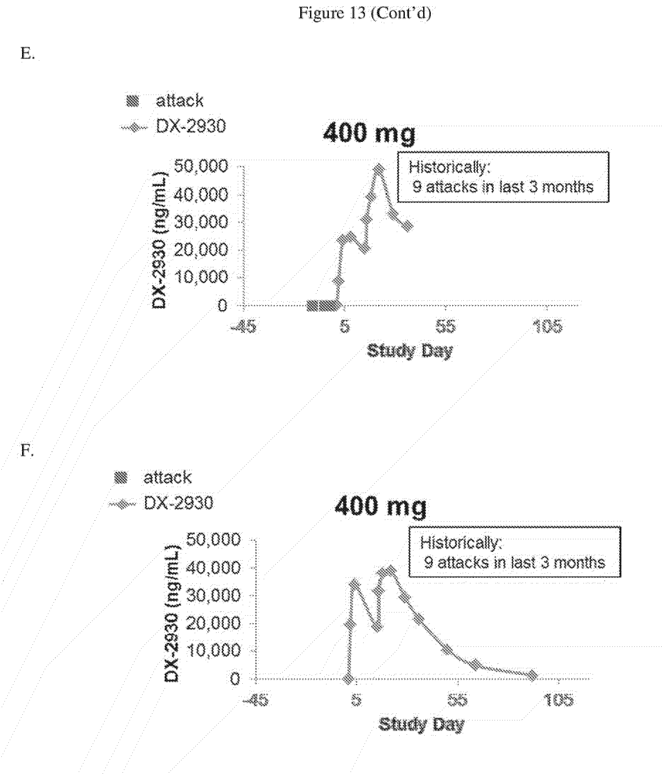

In the placebo subject, attacks occurred throughout the observation period at a high rate (Figure 13, panel A). In contrast, the subject treated with 300 mg DX-2930 was attack-free when drug levels were high. (Figure 13, panel B).

In the four subjects with high baseline attack rates who were treated with 400 mg DX-2930, all four of these subjects were attack-free during the Day 8 to Day 50 time interval (Figures 13, panels C-F). This included one subject with a very high baseline rate of 36 attacks in the past 3 months. The pattern of attacks in these DX-2930-treated subjects was consistent with that seen in the 300 and 400 mg groups overall. These individuals did not experience any attacks when drug levels were high. Attacks only occurred when drug levels were low, either prior to meaningful drug accumulation or after drug levels declined.

From these data, it was observed that the therapeutic effect of DX-2930 was also evident in HAE patients with high baseline attack rates.

In summary, this study shows (a) that there were no apparent safety signals for DX-2930, (b) that the PK profile was consistent overall with a monoclonal antibody and supported a regimen of dosing once every 2 weeks or even less frequently, (c) that pharmacodynamic data demonstrated that DX-2930 normalizes the aberrant instability of HAE plasma, at least in the context of the kininogen biomarker assays, and (d) that a highly statistically significant finding of HAE attack prevention by DX-2930 was observed. Specifically, in comparison to placebo, there were 100% and 88% reduction in attacks by the 300 and 400 mg DX-2930 treatment groups respectively. This clinical effect was logically associated with drug exposure over time and was also observed in the subset of patients with high baseline attack rates.

These data demonstrate proof of concept for DX-2930 in long-term prophylaxis against HAE attacks.

Example 2 : Pharmacodynamic Effect of DX-2930 on Plasma Kallikrein in **Hereditary** Angioedema Patients

Attacks of hereditary angioedema (HAE) result from uncontrolled contact system activation which generates a burst of plasma kallikrein (pKal) that cleaves high-molecular-weight kininogen (HMWK) to produce 2-chain HMWK and the edema-inducing peptide, bradykinin. DX-2930 is a human monoclonal antibody inhibitor of pKal in development for the prevention of HAE attacks. The pharmacodynamic bioactivity of DX-2930 was assessed in subjects with HAE.

As described in Example 1 above, the phase 1b multi-center, double-blind study, randomized subjects with Type 1 or 2 HAE to receive 2 subcutaneous doses of DX-2930 in dose groups of 30, 100, 300 or 400 mg (n=4, 4, 5, 11) or placebo (n=13). Blood samples were obtained prior to and following administration of study drug (Days 1, 8, 22, 64, 92, 120). The ability of DX-2930 to inhibit pKal in basal and FXIIA-activated citrated plasma was assessed using Western blot for 2-chain HMWK.

The results obtained from this study showed that mean 2-chain HMWK levels were significantly reduced and essentially normalized in the 300 and 400 mg dose groups on Days 8 and 22, and on Days 8, 22 and 50, respectively, when compared to placebo treated subjects. Treatment with 300 or 400 mg DX-2930 also attenuated the burst in 2-chain generation to levels at or below that observed in healthy individuals in FXIIA-activated samples. Levels of 2-chain HMWK did not differ from pre-dose plasma samples in either activated or inactivated samples collected on Days 64, 92 or 120 following DX-2930, which correspond to periods of low drug exposure.

In sum, this study indicates that DX-2930 inhibits pKal in a dose and time-dependent manner in HAE patients.

Example 3 : Relationship between Drug Exposure and Clinical Response Observed in the Phase 1b Study of DX-2930 in Subjects with Hereditary Angioedema

DX-2930 is a human monoclonal antibody inhibitor of plasma kallikrein in development for the prevention of hereditary angioedema (HAE) attacks. Data from the phase 1b study of DX-2930 in HAE subjects as described in Example 1 above was analyzed to characterize the relationship between drug exposure and clinical response.

This phase 1b multi-center, double-blind study, randomized subjects with Type 1 or 2 HAE to receive 2 subcutaneous doses of DX-2930 in dose groups of 30, 100, 300 or 400 mg (n=4, 4, 5, 11) or placebo (n=13). In this post-hoc analysis, the incidence of HAE attacks was evaluated in relation to drug exposure over time. In addition, a post-hoc modified intent-to-treat efficacy analysis (MITT) was conducted to assess clinical effect in the context of subjects receiving the full dose regimen of DX-2930.

Placebo-treated subjects reported HAE attacks throughout the study (9 subjects, 65 HAE attacks). In the 300 and 400 mg dose groups, HAE attacks were reported prior to or just after initial dosing. When drug levels were high (Day 8 to 50), all but 1 subject was attack-free. As drug levels waned, attacks re-emerged. In the MITT efficacy analysis, from Day 8 to 50 in comparison to placebo, the 300 and 400 mg DX-2930 groups had a 100% (P < 0.0001) and 95% (P = 0.0022) reduction in attacks, respectively.

Thus, this study indicates that HAE attacks were substantially decreased or were eliminated during periods of notable drug exposure consistent with the suggestion that higher drug levels should correlate with HAE attack prevention.

Example 4 : Modeling and Analyses to Identify Potential Dosing Regimens of DX-2930 for the Long-Term Prophylaxis of Hereditary Angioedema

DX-2930 is a human monoclonal antibody inhibitor of plasma kallikrein in development for the prevention of hereditary angioedema (HAE) attacks. Data from the Phase 1 studies of DX-2930 as described in Example 1 above were modeled and analyzed to identify potential dosing regimens.

Pharmacokinetic, pharmacodynamic and efficacy data from the Phase 1 clinical studies were examined. The incidence of HAE attacks was evaluated in relation to plasma drug concentrations to estimate steady-state trough drug levels necessary to prevent attacks.

Dosing regimens of 300 mg DX-2930 every 2 (q2) or 4 (q4) weeks, and 150 mg q4 weeks are being considered for the pivotal efficacy study. Pharmacokinetic modeling predicts steady-state trough plasma concentrations of 27,000, 9,500, and 4,750 ng/mL, respectively. In the Phase 1b study, at 27,000 ng/mL (corresponding to approximate drug levels at Day 22 for 300 mg DX-2930), 2-chain high-molecular-weight kininogen was suppressed to a level approximating that observed in healthy subjects. Three-hundred mg q2 is therefore predicted to normalize the instability of HAE plasma at steady state. As successful HAE prophylaxis may not require such a high level of pharmacodynamic effect, an analysis of clinical effect in relation to plasma drug concentrations was also conducted. In the Phase 1b study following DX-2930 treatment, 24/25 attacks (96%), 21/25 (84%), and 18/25 attacks (72%) occurred below plasma concentrations of 27,000, 9,500, and 4,750 ng/mL, respectively, suggesting a meaningful range of clinical response is associated with this range of drug exposure.

In this analysis, potential dosing regimens of DX-2930 were identified for further clinical investigation in the pivotal efficacy study.

**Example 5 : Hereditary Angioedema is Associated with Neuropathic Pain, Systemic Lupus Erythematosis and Systemic Mastocytosis in an Analysis of a Health Analytics Claims Database**

The plasma kallikrein kinin system (KKS) has been associated with a variety of diseases in addition to being a key mediator of hereditary angioedema (HAE). It was explored in this study whether HAE patients were predisposed to develop such KKS associated diseases: abdominal aortic aneurysm, anaphylaxis, cardiac vasoplegia syndrome, Crohn's disease, diabetic macular edema, idiopathic anaphylaxis, neuropathic pain, psoriasis, psoriatic arthritis, retinopathy, rheumatoid arthritis, systemic lupus erythematosus (SLE), system mastocytosis, systemic vasculitis, thrombotic cerebrovascular accident, and ulcerative colitis.

The Truven MarketScan Database containing individual-level claims data from medical payers and Medicare supplemental plans for 80 million lives in the U.S. from 1/2010 through 7/2014 was utilized in this study. Within this dataset, an HAE population (n=1063) and 2 control populations: an angioedema population (n=138,851) and the general population (n=79,971,098) exclusive of HAE patients were defined using a combination of ICD-9 and prescription drug codes. Claims for comorbid diseases were identified and compared across the populations with calculated odds ratios and 95% confidence intervals (CI).

As shown in the Table 8 below, in the HAE population, SLE was observed 2.3 times more often (OR 2.30, 95%CI: 1.47-3.59) than the angioedema control population. Neuropathic pain was observed 1.45 times (OR 1.45, 95%CI: 1.01-2.09) and systemic mastocytosis 4.79 times (OR 4.79, 95%CI: 1.51-15.18) more often than the angioedema control population.

## Table 8. Association between HAE and Other KSS-related Diseases

| Associated Disease | HAE Population | | Angioedema Control | | Odds Ratio (95% CI) |
|---|---|---|---|---|---|
| | N=1063 | % | N=138,851 | % | |
| Abnormal aortic aneurysm (AAA) | 6 | 0.56 | 583 | 0.42 | 1.35 (0.60, 3.02) |
| Anaphylaxis | 86 | 8.09 | 15,293 | 11.01 | 0.72 (0.57, 0.89) |
| Cardiac vasoplegia syndrome | 2 | 0.19 | 88 | 0.06 | 2.97 (0.73, 12.09) |
| Crohn's disease | 6 | 0.56 | 848 | 0.61 | 0.92 (0.41, 2.07) |
| Diabetic macular edema | 2 | 0.19 | 140 | 0.10 | 1.87 (0.46, 7.55) |
| Idiopathic anaphylaxis | 59 | 5.55 | 8,442 | 6.08 | 0.91 (0.70, 1.18) |
| Neuropathic pain | 30 | 2.82 | 2,726 | 1.96 | 1.45 (1.01, 2.09) |
| Psoriasis | 14 | 1.32 | 2,182 | 1.57 | 0.84 (0.49, 1.42) |
| Psoriatic arthritis | 4 | 0.38 | 382 | 0.28 | 1.37 (0.51, 3.67) |
| Retinopathy | 2 | 0.19 | 85 | 0.06 | 3.08 (0.76, 12.52) |
| Rheumatoid arthritis | 30 | 2.82 | 3,093 | 2.23 | 1.27 (0.89, 1.84) |
| Systemic lupus erythematosus | 20 | 1.88 | 1,148 | 0.83 | 2.30 (1.47, 3.59) |
| Systemic mastocytosis | 3 | 0.28 | 82 | 0.06 | 4.79 (1.51, 15.18) |
| Systemic vasculitis | 2 | 0.19 | 189 | 0.14 | 1.38 (0.34, 5.58) |
| Thrombo cerebrovasc accident (CVA) | 2 | 0.19 | 375 | 0.27 | 0.70 (0.17, 2.80) |
| Ulcerative colitis | 9 | 0.85 | 902 | 0.65 | 1.31 (0.68, 2.53) |

In sum, an analysis of a large longitudinal claims database revealed that comorbid diseases of SLE, neuropathic pain and systemic mastocytosis had a greater representation in HAE patients than other types of angioedema, suggesting that activity of the KKS may be contributing to the manifestations of these diseases. Accordingly, patients having or at risk for HAE may be predisposed to KKS-associated diseases, including neuropathic pain, systemic lupus erythrmatosus, and systemic mastocytosis. Thus, treatment with a pKal inhibitor, such as DX-2930 may reduce the risk for the development of such a KKS-associated disease.

**Example 6: A Double-Blind Study including a Load Period and a Maintenance Period**

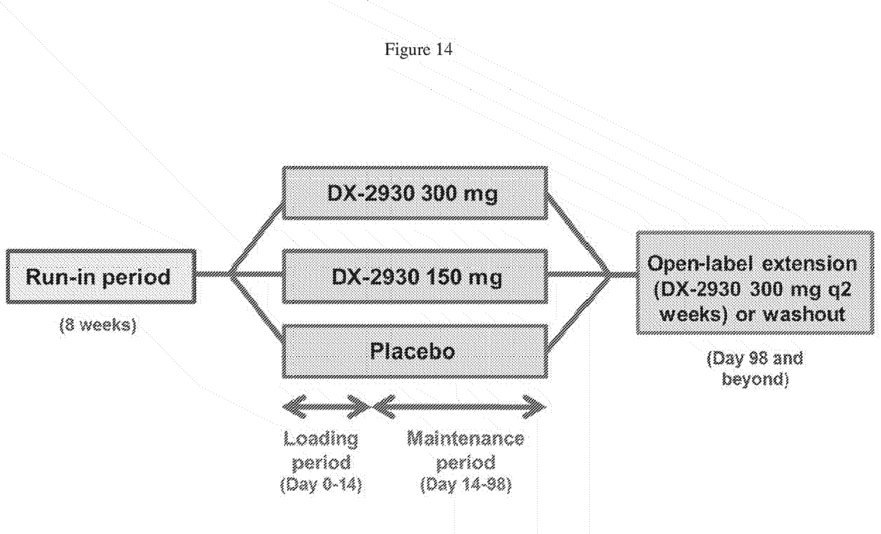

A randomized, double-blind, placebo-controlled, parallel arm study is carried out. Patients are selected for having HAE type I or II patients with at least 1 attack per 4 weeks. A run-in period is used to evaluate baseline HAE attack rate. The patients are subjected to a 1:1:1 randomization into 3 different treatment arms (300 mg DX-2930, 150 mg DX-2930, or placebo) which are administered by subcutaneous injection. The study is designed with a load period and a maintenance period (Figure 14). Subjects are treated on Days 0, 7, and 14 during the loading period, followed by dosing every 2 weeks during the maintenance period (Days 28, 42, 56, 70, and 84).

OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. Thus, other embodiments are also within the claims.

EQUIVALENTS

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of examples only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03.

**The invention further provides the following numbered embodiments**

1. A method of preventing hereditary angioedema (HAE) attack or reducing the rate of HAE attack, the method comprising:
administering to a subject in need thereof about 100 mg to about 400 mg of an antibody, which binds to the same epitope as DX-2930 or competes against DX-2930 for binding to active plasma kallikrein, wherein the antibody is administered every two to four weeks for at least two times.

2. The method of embodiment 1, wherein the antibody comprises the same heavy chain and light chain complementary determining regions (CDRs) as DX-2930.

3. The method of embodiment 1 or 2, wherein the antibody is a full length antibody or an antigen-binding fragment thereof.

4. The method of embodiment 3, wherein the antibody is DX-2930.

5. The method of any one of embodiments 1 to 4, wherein the antibody is administered subcutaneously.

6. The method of any one of embodiments 1 to 5, wherein the subject is a human patient having type I or type II HAE.

7. The method of any one of embodiments 1 to 6, wherein the subject is a human patient experiencing at least two HAE attacks per year prior to the treatment.

8. The method of embodiment 7, wherein the human patient had at least one HAE attack within 6 months prior to the first administration.

9. The method of embodiment 8, wherein the human patient had at least 2 HAE attacks within 3 months prior to the first administration.

10. The method of embodiment 9, wherein the human patient had at least 9 HAE attacks within 3 months prior to the first administration.

11. The method of any one of embodiments 1 to 10, wherein the antibody is administered to the subject at about 100 mg to about 300mg once every two to four weeks.

12. The method of any one of embodiments 1 to 11, wherein the antibody is administered to the subject at 300 mg once every two to four weeks.

13. The method of any one of embodiments 1 to 10, wherein the antibody is administered to the subject at least two times, which are two to four weeks apart, and wherein one is at 300 mg and the other is at 400 mg.

14. The method of any one of embodiments 1 to 13, wherein the antibody is administered to the subject every two weeks.

15. The method of any one of embodiments 1 to 13, wherein the antibody is administered to the subject every four weeks.

16. The method of any one embodiments of 1 to 15, wherein administration of the antibody reduces cleaved kininogen of the subject to a level comparable to healthy subjects.

17. The method of any one of embodiments 1 to 16, wherein the antibody is formulated in a pharmaceutical composition comprising a pharmaceutically acceptable carrier.

18. The method of embodiment 17, wherein the composition comprises sodium phosphate, citric acid, histidine, sodium chloride, and Tween 80.

19. The method of any one of embodiments 1-18, wherein the antibody is formulated in 30 mM sodium phosphate, 8.6 mM citric acid, 50 mM histidine, 90 mM sodium chloride, and 0.01% Tween 80, pH 6.0.

20. A method of treating hereditary angioedema (HAE), the method comprising:

administering to a subject in need thereof an antibody that binds to the same epitope as DX-2930 or competes against DX-2930 for binding to active plasma kallikrein, the antibody being first administered for a loading period, and then for a maintenance period;
wherein in the loading period, the antibody is administered once every week, and wherein in the maintenance period, the antibody is administered once every two to four weeks.

21. The method of embodiment 20, the antibody comprises the same CDRs as DX-2930.

22. The method of embodiment 20 or 21, wherein the antibody is a full length antibody or an antigen-binding fragment thereof.

23. The method of embodiment 22, wherein the antibody is DX-2930.

24. The method of any one of embodiments 20-23, wherein the antibody is administered subcutaneously.

25. The method of any one of embodiments 20-24, wherein the subject is a human patient having, suspected of having, or at risk for HAE.

26. The method of any one of embodiments 20-25, wherein the antibody is administered for preventing HAE attack or reducing the rate of HAE attack.

27. The method of any one of embodiments 20-26, wherein the subject is a human patient who had experienced at least two HAE attacks per year prior to the treatment.

28. The method of any one of embodiments 20-27, wherein in the loading period, the antibody is administered to the subject at 100 to 300 mg.

29. The method of embodiment 28, wherein the antibody is administered to the subject at 150 mg or 300 mg.

30. The method of any one of embodiments 20-29, wherein the loading period is two weeks and the antibody is administered at day 0, day 7, and day 14.

31. The method of any one of embodiments 20-30, wherein in the maintenance period, the antibody is administered to the subject at 100 to 300 mg.

32. The method of embodiment 31, wherein the antibody is administered to the subject at 150 mg or 300 mg.

33. The method of any one of embodiments 20-32, wherein the maintenance period is 10 weeks and the antibody is administered at day 28, day 42, day 56, day 70, and day 84.

34. The method of any one of embodiments 20-33, wherein the method further comprises administering to the subject the antibody after the maintenance period once every two to four weeks.

35. The method of embodiment 34, wherein the antibody is administered at 100 to 300 mg.

36. The method of embodiment 35, wherein the antibody is administered at 300 mg.

37. The method of any one of embodiments 20-36, wherein the subject has HAE type I or type II.

38. The method of any one of embodiments 20-37, wherein the subject has at least one attack every four weeks prior to the first administration.

39. The method of any one of embodiments 20-38, wherein the antibody is formulated in a pharmaceutical composition comprising a pharmaceutically acceptable carrier.

40. The method of embodiment 39, wherein the pharmaceutically composition comprises sodium phosphate, citric acid, histidine, sodium chloride, and Tween 80.

41. The method of embodiment 40, wherein the antibody is formulated in in 30 mM sodium phosphate, 8.6 mM citric acid, 50 mM histidine, 90 mM sodium chloride, and 0.01% Tween 80.

**Claims**

1. A pharmaceutical composition comprising an antibody and a pharmaceutically acceptable carrier for use in preventing hereditary angioedema (HAE) attack or reducing the rate of HAE attack in a subject in thereof:

   wherein the antibody comprises a heavy chain complementarity determining region (HCDR) 1 set forth as HYIMM (SEQ ID NO: 5), a HCDR2 set forth as GIYSSGGITVYADSVKG (SEQ ID NO: 6), a HCDR3 set forth as RRIGVPRRDEFDI (SEQ ID NO: 7), a light chain complementarity determining region (LCDR) 1 set forth as RASQSISSWLA (SEQ ID NO: 8), a LCDR2 set forth as KASTLES (SEQ ID NO: 9), and a LCDR3 set forth as QQYNTYWT (SEQ ID NO: 10);
   and wherein the pharmaceutical composition comprises sodium phosphate, citric acid, histidine, sodium chloride, and Tween 80.

2. The pharmaceutical composition for use of claim 1, wherein the antibody comprises
   a heavy chain variable domain set forth as

   EVQLLESGGGLVQPGGSLRLSCAASGFTFSHYIMMWVRQAPGKGLEWVSGIYSSGGITV
   YADSVKGRFTISRDNSKNTLYLQMNSLRAEDTAVYYCAYRRIGVPRRDEFDIWGQGTM
   VTVSS (SEQ ID NO: 3), and

   a light chain variable domain set forth as

   DIQMTQSPSTLSASVGDRVTITCRASQSISSWLAWYQQKPGKAPKLLIYKASTLESGVPS
   RFSGSGSGTEFTLTISSLQPDDFATYYCQQYNTYWTFGQGTKVEIK (SEQ ID NO: 4).

3. The pharmaceutical composition for use of claim 1 or claim 2, wherein the antibody is a full-length antibody or an antigen-binding fragment thereof.

4. The pharmaceutical composition for use of claim 3, wherein the antibody comprises
   a heavy chain set forth as

   EVQLLESGGGLVQPGGSLRLSCAASGFTFSHYIMMWVRQAPGKGLEWVSGIYSSGGITV
   YADSVKGRFTISRDNSKNTLYLQMNSLRAEDTAVYYCAYRRIGVPRRDEFDIWGQGTM
   VTVSSASTKGPSVFPLAPSSKSTSGGTAALGCLVKDYFPEPVTVSWNSGALTSGVHTFPA

VLQSSGLYSLSSVVTVPSSSLGTQTYICNVNHKPSNTKVDKRVEPKSCDKTHTCPPCPAP
ELLGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVY
TLPPSREEMTKNQVSLTCLVKGFYPSDIAVEWESNGQPENNYKTTPPVLDSDGSFFLYSK
LTVDKSRWQQGNVFSCSVMHEALHNHYTQKSLSLSPG, and

a light chain set forth as

DIQMTQSPSTLSASVGDRVTITCRASQSISSWLAWYQQKPGKAPKLLIYKASTLESGVPS
RFSGSGSGTEFTLTISSLQPDDFATYYCQQYNTYWTFGQGTKVEIKRTVAAPSVFIFPPSD
EQLKSGTASVVCLLNNFYPREAKVQWKVDNALQSGNSQESVTEQDSKDSTYSLSSTLTL
SKADYEKHKVYACEVTHQGLSSPVTKSFNRGEC.

5. The pharmaceutical composition for use of any one of claims 1-4, wherein the antibody is administered subcutaneously.

6. The pharmaceutical composition for use of any one of claims 1-5, wherein the antibody is administered at about 100 mg to about 400 mg every two to four weeks for at least two times.

7. The pharmaceutical composition for use of any one of claims 1-5, wherein:

    (a) the antibody is administered to the subject at about 100 mg to about 300 mg once every two to four weeks;
    (b) the antibody is administered to the subject at 300 mg once every two weeks; or
    (c) the antibody is administered to the subject at 300 mg once every four weeks.

8. The pharmaceutical composition for use of any one of claims 1-5, wherein:

    wherein the antibody is first administered for a loading period, and then for a maintenance period; and
    wherein in the loading period, the antibody is administered once every week, and wherein in the maintenance period, the antibody is administered at 300mg once every two to four weeks.

9. The pharmaceutical composition for use of claim 8, wherein

    (a) in the loading period, the antibody is administered to the subject at 100 to 300 mg, optionally wherein the antibody is administered to the subject at 150 mg or 300 mg;
    (b) the loading period is two weeks and the antibody is administered at day 0, day 7, and day 14; and/or
    (c) the maintenance period is 10 weeks and the antibody is administered at day 28, day 42, day 56, day 70, and day 84.

10. The pharmaceutical composition for use of any one of claims 1-9, wherein the subject is a human patient having, suspected of having, or at risk for HAE, optionally wherein the HAE is type I HAE or type II HAE.

11. The pharmaceutical composition for use of any one of claims 1-10, wherein the subject is a human patient experiencing at least two HAE attacks per year prior to the treatment, optionally wherein the human patient had at least two HAE attacks within 3 months prior to the first administration, and preferably wherein the human patient had at least 9 HAE attacks within 3 months prior to the first administration.

12. The pharmaceutical composition for use of claim 11, wherein the subject is a human patient who had at least one HAE attack within 6 months prior to the first administration.

13. The pharmaceutical composition for use of any one of claims of 1-12, wherein administration of the antibody reduces cleaved kininogen of the subject to a level comparable to healthy subjects.

14. The pharmaceutical composition for use of any one of claims 1-13, wherein the pharmaceutical composition comprises 30 mM sodium phosphate, 8.6 mM citric acid, 50 mM histidine, 90 mM sodium chloride, and 0.01% Tween 80, optionally pH 6.0.

Figure 1

Figure 2

Figure 3

Figure 4

Figure 5

Figure 6

A.

B.

Figure 7

Figure 8

Figure 9

Figure 10

Figure 11

Figure 12

Figure 13

A.

B.

Figure 13 (Cont'd)

C.

D.

Figure 13 (Cont'd)

E.

F.

Figure 14

Europäisches
Patentamt

European
Patent Office

Office européen
des brevets

**EUROPEAN SEARCH REPORT**

Application Number

EP 21 16 7802

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/152232 A2 (DYAX CORP [US]) 25 September 2014 (2014-09-25) * page 26, line 28 - page 27, line 10; sequences 46,47 * * page 28, line 27 - page 29, line 21 * * page 16 - page 17 * | 1-14 | INV. C07K16/40 |
| X | CHYUNG YUNG ET AL: "A phase 1 study investigating DX-2930 in healthy subjects", ANNALS OF ALLERGY, ASTHMA & IMMUNOLOGY, ARLINGTON HEIGHTS, IL, US, vol. 113, no. 4, 26 June 2014 (2014-06-26), page 460, XP029068455, ISSN: 1081-1206, DOI: 10.1016/J.ANAI.2014.05.028 * page 461, column 1 - column 2 * * page 462, column 1 * * abstract * | 1-14 | |
| X | Dyax: "Dyax Announces Positive Results from Phase 1a Clinical Trial of DX-2930", dyax, 25 February 2014 (2014-02-25), pages 1-3, XP055317760, Retrieved from the Internet: URL:http://files.shareholder.com/downloads/DYAX/0x0x728704/080fce37-bdbe-4c1b-beaf-59632ab0ae15/DYAX_News_2014_2_25_Earnings.pdf [retrieved on 2016-11-09] * page 1 * | 1-14 | TECHNICAL FIELDS SEARCHED (IPC) A61K C07K |

-/--

The present search report has been drawn up for all claims

| Place of search | Date of completion of the search | Examiner |
|---|---|---|
| Munich | 17 October 2021 | Saame, Tina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

53

Europäisches
Patentamt

European
Patent Office

Office européen
des brevets

## EUROPEAN SEARCH REPORT

Application Number

EP 21 16 7802

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | J. A. KENNISTON ET AL: "Inhibition of Plasma Kallikrein by a Highly Specific Active Site Blocking Antibody", JOURNAL OF BIOLOGICAL CHEMISTRY, vol. 289, no. 34, 26 June 2014 (2014-06-26), pages 23596-23608, XP055215056, ISSN: 0021-9258, DOI: 10.1074/jbc.M114.569061 * the whole document * | 1-14 | |

-----

TECHNICAL FIELDS
SEARCHED        (IPC)

The present search report has been drawn up for all claims

| Place of search | Date of completion of the search | Examiner |
|---|---|---|
| Munich | 17 October 2021 | Saame, Tina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 16 7802

This annex lists the patent family members relating to the patent documents cited in the above-mentioned European search report.
The members are as contained in the European Patent Office EDP file on
The European Patent Office is in no way liable for these particulars which are merely given for the purpose of information.

17-10-2021

| Patent document cited in search report | Publication date | Patent family member(s) | Publication date |
|---|---|---|---|
| WO 2014152232 A2 | 25-09-2014 | AU 2014240045 A1 | 10-09-2015 |
| | | AU 2019200638 A1 | 21-02-2019 |
| | | AU 2021200144 A1 | 18-03-2021 |
| | | BR 112015022282 A2 | 10-10-2017 |
| | | CA 2906624 A1 | 25-09-2014 |
| | | CN 105051068 A | 11-11-2015 |
| | | CN 111704672 A | 25-09-2020 |
| | | EP 2970502 A2 | 20-01-2016 |
| | | EP 3594244 A1 | 15-01-2020 |
| | | HK 1218762 A1 | 10-03-2017 |
| | | JP 2016513682 A | 16-05-2016 |
| | | JP 2020007354 A | 16-01-2020 |
| | | KR 20150132473 A | 25-11-2015 |
| | | US 2016017055 A1 | 21-01-2016 |
| | | US 2019185580 A1 | 20-06-2019 |
| | | WO 2014152232 A2 | 25-09-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62140277
- US 62214293
- US 62140289
- US 5260203 A
- US 4946778 A
- US 4881175 A
- US 6407213 B
- US 5693762 A
- US 5565332 A
- US 5580717 A
- US 5733743 A
- US 6265150 B
- WO 8704462 A
- US 4704692 A
- US 4399216 A
- US 4634665 A
- US 5179017 A
- US 5849992 A
- US 4522811 A
- US 5374548 A
- US 5399331 A
- WO 9521601 A
- WO 2003103475 A


**Non-patent literature cited in the description**

- **SAINZ I.M. et al.** *Thromb Haemost,* 2007, vol. 98, 77-83
- **ZURAW B.L. et al.** *N Engl J Med,* 2008, vol. 359, 1027-1036
- **DE WILDT et al.** *Eur J Immunol.,* 1996, vol. 26 (3), 629-39
- **KABAT, E.A. et al.** Sequences of Proteins of Immunological Interest. U.S. Department of Health and Human Services, NIH Publication, 1991
- **CHOTHIA, C. et al.** *J. Mol. Biol.,* 1987, vol. 196, 901-917
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **BOWIE et al.** *Science,* 1990, vol. 247, 1306-1310
- **KARLIN ; ALTSCHUL.** *Proc. Natl. Acad. Sci. USA,* 1990, vol. 87, 2264-68
- **KARLIN ; ALTSCHUL.** *Proc. Natl. Acad. Sci. USA,* 1993, vol. 90, 5873-77
- **ALTSCHUL et al.** *J. Mol. Biol.,* 1990, vol. 215, 403-10
- **ALTSCHUL et al.** *Nucleic Acids Res.,* 1997, vol. 25 (17), 3389-3402
- **HARLOW ; LANE.** Antibodies: A Laboratory Manual. Cold Spring Harbor Laboratory, 1988
- **GREENFIELD.** Antibodies: A Laboratory Manual. Cold Spring Harbor Laboratory Press, 2013
- **WINTER et al.** *Annu. Rev. Immunol.,* 1994, vol. 12, 433-455
- **MCCAFFERTY et al.** *Nature,* 1990, vol. 348, 552-553
- **MORRISON et al.** *Proc. Nat. Acad. Sci.,* 1984, vol. 81, 6851
- **MORRISON et al.** *Proc. Natl. Acad. Sci. USA,* 1984
- **NEUBERGER et al.** *Nature,* 1984, vol. 312, 604
- **TAKEDA et al.** *Nature,* 1984, vol. 314, 452
- **QUEEN et al.** *Proc. Natl. Acad. Sci. USA,* 1989, vol. 86, 10029-10033
- **NADKARNI, A. et al.** *Protein Expr Purif,* 2007, vol. 52 (1), 219-29
- **POWERS et al.** *J. Immunol. Methods,* 2001, vol. 251, 123-35
- **SCHOONOOGHE S. et al.** *BMC Biotechnol.,* 2009, vol. 9, 70
- **ABDEL-SALAM, HA. et al.** *Appl Microbiol Biotechnol,* 2001, vol. 56 (1-2), 157-64
- **TAKAHASHI K. et al.** *Biosci Biotechnol Biochem,* 2000, vol. 64 (10), 2138-44
- **EDQVIST, J. et al.** *J Biotechnol,* 1991, vol. 20 (3), 291-300
- **BAUMANN K. et al.** *BMC Syst. Biol.,* 2010, vol. 4, 141
- **DRAGOSITS, M. et al.** *BMC Genomics,* 2010, vol. 11, 207
- **DRAGOSITS, M. et al.** *J Proteome Res.,* 2009, vol. 8 (3), 1380-92
- **NING, D. et al.** *J. Biochem. and Mol. Biol.,* 2005, vol. 38 (3), 294-299
- **KOZYR, AV et al.** *Mol Biol (Mosk),* 2004, vol. 38 (6), 1067-75
- **HORWITZ, AH. et al.** *Proc Natl Acad Sci U S A,* 1988, vol. 85 (22), 8678-82

- **BOWDISH, K. et al.** *J Biol Chem,* 1991, vol. 266 (18), 11901-8
- **GASSER, B. et al.** *Biotechol. Bioeng.,* 2006, vol. 94 (2), 353-61
- **KOBAYASHI H. et al.** *FEMS Microbiol Lett,* 1997, vol. 152 (2), 235-42
- **KO JH. et al.** *Appl Biochem Biotechnol,* vol. 60 (1), 41-8
- **SMITH, BJ. et al.** *Bioconjug Chem,* 2001, vol. 12 (5), 750-756
- **URLAUB ; CHASIN.** *Proc. Natl. Acad. Sci. USA,* 1980, vol. 77, 4216-4220
- **KAUFMAN ; SHARP.** *Mol. Biol.,* 1982, vol. 159, 601-621
- *J. Immunol. Methods,* 2004, vol. 289 (1-2), 65-80

- **GALEFFI, P. et al.** *J Transl Med,* 2006, vol. 4, 39
- **BURTON ; WOOF.** *Adv. Immunol.,* 1992, vol. 51, 1-84
- **JEFFERIS et al.** *Immunol. Rev.,* 1998, vol. 163, 59-76
- **BERGE, S.M. et al.** *J. Pharm. Sci.,* 1977, vol. 66, 1-19
- Sustained and Controlled Release Drug Delivery Systems. Marcel Dekker, Inc, 1978
- **V.V. RANADE.** *J. Clin. Pharmacol.,* 1989, vol. 29, 685
- **KAPLAN, A.P.** *J Allergy Clin Immunol,* 2010, vol. 126 (5), 918-925
- **MCMILLAN, C.V. et al.** *Patient,* 2012, vol. 5 (2), 113-26